# FASCICULE DE BREVET EUROPEEN

(11) **EP 3 362 481 B1**
(45) Date de publication et mention de la délivrance du brevet: **08.07.2026**
(21) Numéro de dépôt: 16782218.8
(22) Date de dépôt: 14.10.2016
(51) Int. Cl.: C07K 16/34, C12P 21/02, C12N 15/00, C07K 14/705

(54) **PROCEDE DE PRODUCTION DE PROTEINES ERYTHROCYTAIRES**
VERFAHREN ZUR HERSTELLUNG VON ERYTHROZYTENPROTEINEN
METHOD FOR PRODUCING ERYTHROCYTE PROTEINS

(30) Priorité: 16.10.2015 FR 1559895
(43) Date de publication de la demande: 22.08.2018
(73) Titulaire: Institut National Transfusion Sanguine, 75739 Paris Cedex 15 (FR); Institut National de la Santé et de la Recherche Médicale (INSERM), 75013 Paris (FR); Université Paris Cité, 75006 Paris (FR)
(72) Inventeur: MOURO-CHANTELOUP, Isabelle, 92210 Saint Cloud (FR); GENETET, Sandrine, 94800 Villejuif (FR)
(74) Mandataire: Regimbeau
(86) Numéro de dépôt international: PCT/EP2016/074792
(87) Numéro de publication internationale: WO 2017/064294

(56) Documents cités:
- WO-A1-2007/038755
- WO-A1-2012/095872
- WO-A1-2015/095854
- WO-A2-00/32632
- WO-A2-2005/081743
- WO-A2-2008/021290
- WO-A2-2008/106660
- WO-A2-99/37763
- BERNHARD FRANK ET AL: "Funktionale Membranproteine durch optimierte Lipidumgebung in Nanodiscs", BIOSPEKTRUM, SPEKTRUM AKADEMISCHER VERLAG, DE, vol. 21, no. 6, 1 October 2015 (2015-10-01), pages 640 - 642, XP035551633, ISSN: 0947-0867, [retrieved on 20151001], DOI: 10.1007/S12268-015-0626-3

## Description

### INTRODUCTION

Les groupes sanguins sont déterminés par un ensemble d'antigènes de surface qui sont regroupés en systèmes, sur des critères génétiques. Aujourd'hui on dénombre 35 systèmes de groupes sanguins (ABO, Rhésus, Kell, Duffy, MNS...) définissant plus de 300 antigènes érythrocytaires. Tandis que certains ont une large distribution tissulaire, comme ABO, d'autres, comme Rhésus (RH), sont spécifiques aux globules rouges. Certains antigènes portés par les érythrocytes sont fortement immunogènes, en particulier ceux des systèmes RH et Kell.

Le système Rhésus (RH) est le système de groupe sanguin le plus complexe, car le plus polymorphe et le plus immunogène sur le plan transfusionnel. Le phénotype RH courant comprend l'antigène majeur RH1 (D) codé par le gène RHD, et les antigènes RH2 (C), RH3 (E), RH4 (c) et RH5 (e) codés par le gène RHCE. En fonction des formes alléliques, 8 haplotypes classiques sont distingués.

Le système RH joue un rôle important en médecine transfusionnelle. En effet, ses antigènes peuvent être, dans des conditions d'incompatibilités de groupe sanguin, à l'origine de développement d'allo-anticorps chez des receveurs ou la femme enceinte, entrainant alors des accidents hémolytiques et/ou la Maladie Hémolytique du Nouveau-Né (MHNN). L'allo-immunisation anti-D correspond, chez la femme enceinte RhD négatif, à la synthèse d'anticorps IgG anti-D en réponse au passage transplacentaire d'hématies fœtales RhD positif dans la circulation maternelle. Les anti-D maternels traversant le placenta vers la circulation fœtale provoquent en retour une hémolyse et une anémie chez le fœtus RhD positif.

Aujourd'hui, si le développement éventuel d'anticorps anti-D dans la plupart des situations d'incompatibilité peut être le plus souvent évité par des tests de détection performants et par une prophylaxie efficace chez les femmes enceintes RHD-négatives, il existe encore des situations d'allo-immunisation. En particuliers, certains sujets de phénotype D-positif peuvent développer des allo-anticorps anti-D dirigés contre un ou plusieurs épitopes manquants, définissant les phénotypes « D partiel », que l'on distingue selon la présence ou l'absence d'un ou plusieurs épitopes D localisés dans les boucles extracellulaires de la protéine RHD (Cartron et al, 1996). L'antigène D est donc un antigène complexe, car il est composé d'une mosaïque d'épitopes, dont au moins neuf (epD1 à epD9) ont été définis à l'aide d'une batterie d'anticorps monoclonaux (Tippett et al, 1996).

Actuellement, l'identification d'anticorps plasmatiques dirigés contre les antigènes RH nécessite l'utilisation de panels constitués d'hématies-test exprimant différents phénotypes RH. Si la plupart de ces phénotypes sont fréquents, certains correspondent à des groupes sanguins rares, dont la disponibilité peut constituer un réel problème, rendant parfois difficile l'identification des anticorps dirigés contre ces antigènes. En outre, l'utilisation de panels implique un risque de présence d'agents infectieux dans l'échantillon.

C'est dans un contexte de sécurité transfusionnelle d'identification d'allo-anticorps anti-RH présents dans le sérum des patients que nous nous proposons de développer de nouveaux outils permettant leur caractérisation efficace, rapide, et qui ne dépendra pas de la disponibilité de sangs rares. Ainsi, la production de la protéine RhD, et plus globalement de protéine Rh, apparait comme une alternative permettant de pallier ces problèmes de détection des allo-anticorps dirigés contre des antigènes de faible prévalence, voire des antigènes plus répandus.

Depuis la découverte des gènes codant les protéines RH, les bases moléculaires des phénotypes RH ont été identifiées (Mouro-Chanteloup *et al,* 1993) et l'expression des protéines RH recombinantes a été réalisée dans différents systèmes hétérologues. Par ailleurs, il a également été décrit des techniques pour la synthèse de protéines membranaires *in vitro,* telles que l'utilisation de nanodisques (WO2007/038755; WO2005/081743; WO2015/095854), pour solubiliser et stabiliser les protéines membranaires. Pour permettre aux protéines exprimées *in vitro* d'être synthétisées dans une conformation fonctionnelle, la composition lipidique des nanodisques est essentielle. Certains nanodisques, particulièrement des nanodisques commerciaux, ne permettent pas d'obtenir une conformation conservant la structure et/ou l'activité de toutes les protéines membranaires (Bernhard Frank et al. (2015), Contrairement à la synthèse des protéines Rh non-érythrocytaires (RHCG) dont les niveaux d'expression ont permis, après extraction de la membrane plasmique, une reconstitution fonctionnelle dans des protéoliposomes (Mouro-Chanteloup *et al,* 2010), il s'est avéré difficile d'obtenir une expression membranaire significative des protéines RHD et RHCE dans leurs conformations permettant de conserver les épitopes reconnus par les anticorps. En raison de leur organisation oligomérique complexe, ces protéines ne peuvent s'exprimer qu'en présence de la protéine associée RHAG (RH-Associated Glycoprotein) (Cartron *et al,* 1999, Mouro-Chanteloup *et al,* 2002) (Goossens et al., Plos one, 2013). Or celle-ci n'a pu être exprimée qu'à 10000 copie/cellule (10% de son expression sur les globules rouges) dans des systèmes hétérologues. Aussi ces niveaux d'expression ne sont pas compatibles avec une utilisation des protéines RH recombinantes comme outil de détection d'anticorps dans le sérum de patients, en particuliers des anticorps de faible titre ou de faible affinité.

Des séquences d'acides aminés des protéines érythrocytaires sont spécifiquement décrites dans certaines références, par exemple : RhCE et RhD (WO 00/32632 et WO 99/37763), RhAG (WO 2008/021290), et UTB (WO 2012/095872) mais aucune de ces références ne décrit une protéine érythrocytaire formulée de manière à être efficacement utilisées en diagnostique.

Il y a donc toujours un besoin pour un système conduisant à l'expression de protéines érythrocytaires dans une configuration native et à des niveaux suffisants pour permettre une utilisation diagnostique.

### DESCRIPTION

### La présente invention est exposée dans le jeu des revendications.

La présente invention a pour objet un système de production de protéines érythrocytaires qui permet d'obtenir de grandes quantités de ces protéines dans leur état natif.

Alors qu'il n'avait jusqu'ici pas été possible de produire des protéines érythrocytaires telles que RhD, RhCE, RhAG et UTB, quel que soit le système utilisé, les inventeurs ont montré qu'il était possible d'obtenir de grandes quantités de ces protéines érythrocytaires dans une conformation leur permettant de conserver les épitopes reconnus par les anticorps, quand elles sont produites *in vitro* en présence de détergents, de liposomes ou de nanodisques, préférentiellement en présence de nanodisques avec une composition lipidique de type POPC.

La méthode optimisée par les inventeurs pour la production acellulaire de protéines érythrocytaires telles que RhD, RhCE, RhAG ou UTB a permis d'obtenir des compositions inédites comprenant les dites protéines en quantité plus importante que ce qui avait été possible avec les méthodes de l'art antérieur. De manière particulièrement surprenant, ladite méthode pour la production acellulaire de protéines érythrocytaires tel que RhD a permis de s'affranchir de l'utilisation de la protéine associée RHAG (RH-Associated Glycoprotein) pour produire ladite protéines RhD contrairement à l'enseignement de l'art antérieur(Mouro-Chanteloup et al., Blood 2002, Goossens et al., Plos one, 2013).

La présente invention a donc pour objet un système acellulaire de production de protéines érythrocytaires telles que RhD, RhCE, RhAG ou UTB caractérisé par la présence de détergents ou de liposomes ou de nanodisques.

Par « protéine érythrocytaire », on entend ici une protéine exprimée dans les cellules de la lignée érythrocytaire. Par « lignée érythrocytaire », on entend ici l'ensemble des types cellulaires dont la différenciation conduit directement ou indirectement aux hématies, y inclus les hématies. La lignée érythrocytaire au sens de l'invention comprend, entre autres, les proérythroblastes, les érythroblastes basophiles, les érythroblastes polychromatophiles, les érythroblastes acidophiles, les réticulocytes et les hématies. Préférablement, une protéine érythrocytaire au sens de l'invention est une protéine qui est exprimée principalement dans la lignée érythrocytaire.

La protéine érythrocytaire de l'invention est en particulier une protéine de la membrane. Ladite protéine peut être une protéine intégrale de la membrane ou une protéine associée à la membrane. De préférence, la protéine érythrocytaire de l'invention est une protéine intégrale de la membrane ; plus préférentiellement, il s'agit d'une protéine polytopique ; encore plus préférentiellement, ladite protéine polytopique porte des antigènes de groupe sanguin.

Parmi les protéines érythrocytaires qui sont particulièrement pertinentes pour la présente invention, on citera les protéines RhD, RhCE, RhAG et UTB et les variants de celles-ci. En effet, il est à l'heure actuelle impossible de produire ces protéines sous forme native en grande quantité. La production dans des systèmes cellulaires de protéines de Rh érythrocytaires est possible mais avec un rendement extrêmement faible et tout à fait insuffisant pour une utilisation en diagnostic. Par ailleurs, la production cellulaire nécessite une co-expression avec RhAG pour l'adressage correct de certaines protéines (e.g. RhD et RhCE). En revanche, la méthode mise au point par les inventeurs de production de protéines polytopiques intégrales de la membrane du globule rouge portant des antigènes de groupe sanguin, c'est-à-dire notamment RhAG, RhD, RhCE et UTB, a permis d'obtenir des compositions inédites comprenant les dites protéines en quantité plus importante que ce qui est possible avec les méthodes de l'art antérieur.

On entend par « protéine RhD » une protéine membranaire non glycosylée de 417 acides aminés, comportant 12 domaines transmembranaires et dont les extrémités Net C-terminales sont intracytoplasmiques. L'antigène D est une collection d'épitopes dépendant de la conformation tout du long de la protéine RhD. Il existe de nombreux anticorps monoclonaux reconnaissant ces épitopes conformationnels de la protéine RhD. Ces anticorps permettent entre autres de vérifier le repliement correct de ladite protéine RhD ou de caractériser les variants de celle-ci (Avent and Reid, 2000). De préférence, la protéine RhD est une protéine ayant la séquence représentée par SEQ ID NO. 5 (NP_001121163). Encore plus préférentiellement, la protéine RhD est codée par le gène RHD dont la séquence est représentée par SEQ ID NO. 1 (NM_001127691).

On entend par « protéine RHCE » une protéine membranaire non glycosylée de 417 acides aminés, comportant 12 domaines transmembranaires et dont les extrémités Net C-terminales sont intracytoplasmiques. De préférence, la protéine RhCE est une protéine ayant la séquence représentée par SEQ ID NO. 6 (NP_065231). Encore plus préférentiellement, la protéine RhCE est codée par le gène RHCE dont la séquence est représentée par SEQ ID NO. 2 (NM_020485). Le gène RHCE présente une très forte homologie avec le gène RHD : ces deux gènes ont environ 96 % d'identité.

La « protéine RhAG » telle qu'on l'entend au sens de l'invention est une protéine membranaire glycosylée de 409 acides aminés, comportant 12 domaines transmembranaires. Cette protéine a une activité de transporteur d'ammonium. De préférence, la protéine RhAG est une protéine ayant la séquence représentée par SEQ ID NO. 7 (NP_000315). Encore plus préférentiellement, la protéine RhAG est codée par le gène RHAG dont la séquence est représentée par SEQ ID NO. 3 (NM_000324).

Par « protéine UTB », on fait ici référence à un transporteur d'urée comportant 10 domaines transmembranaires et dont les extrémités N- et C-terminales sont intracytoplasmiques. La protéine UTB porte les antigènes Kidd. De préférence, la protéine UTB est une protéine ayant la séquence représentée par SEQ ID NO. 8 (NP_001122060). Encore plus préférentiellement, la protéine UTB est codée par le gène SLC14A1 dont la séquence est représentée par SEQ ID NO. 3 (NM_00112858 8).

De nombreux variants phénotypiques sont connus pour chacun de ces loci, et en particulier pour le locus RHD (voir par exemple, Avent and Reid, The Rh blood group system: a review. Blood 95(2):375-387, 2000 ; The Blood Group Antigen FactsBook, 3rd Edition, Ed. : Marion E. Reid, Christine Lomas-Francis, Martin L. Olsson, Academic Press, 2012, ISBN: 978-0-12-415849-8).

On entend ici par « variant » d'un gène ou d'une protéine donnée, un polynucléotide ou un polypeptide dont la séquence présente au moins 95%, préférablement 97 %, plus préférablement 98 %, encore plus préférablement 99 %, d'homologie avec la séquence dudit gène ou de ladite protéine, respectivement. Ces variants peuvent survenir par au moins quatre mécanismes : (1) des réarrangements chromosomiques ; (2) des mutations ponctuelles entrainant un ou des changements d'acide aminé ; (3) des mutations non-sens et (4) des délétions de nucléotides conduisant à l'apparition de codons stop.

Des réarrangements chromosomiques affectant les gènes de protéines érythocytaires de l'invention sont bien connus. Par exemple, les gènes RHCE et RHD qui sont très fortement homologues (plus de 96 % d'identité) sont arrangés en tandem, ce qui facilite les réarrangements géniques entre ces gènes et l'apparition de « gènes hybrides ».

Par ailleurs les gènes RHD et RHCE, présentent un haut degré de polymorphisme. De la même façon, il existe pour chacun des gènes RHAG et SLC14A1 de nombreux variants. De nombreuses mutations dans ces gènes ont été décrites (voir par exemple : The Blood Group Antigen FactsBook, 3rd Edition, Ed. : Marion E. Reid, Christine Lomas-Francis, Martin L. Olsson, Academic Press, 2012, ISBN: 978-0-12-415849-8). On peut aussi se référer au site « Blood Group Antigen Gene Mutation Database » (http://www.ncbi.nlm.nih.gov/gv/rbc/xstcgi.fcgi?cmd=bgmut/systems_info&system= rh) qui compile les différentes mutations observées dans chacun de ces gènes.

La méthode de l'invention permet non seulement de produire chacune des protéines RhD, RhCE et UTB, mais encore l'ensemble des variants de chacune de ces protéines. Dans certains modes de réalisation, l'usage des codons de la séquence nucléotidique codant la protéine érythrocytaire d'intérêt est optimisée pour une utilisation dans des systèmes acellulaires dérivant d'organismes différents de l'homme (par exemple, bactérie telle que *E. coli).* On entend ici par « optimisation des codons » un processus par lequel une séquence nucléotidique codant un polypeptide d'intérêt est modifiée afin d'être optimisée pour l'expression dans un organisme particulier, sans que la séquence d'acides aminés dudit polypeptide ne soit affectée. Un « codon » est une séquence de trois nucléotides traduite en un acide aminé particulier dans une cellule. Il est bien connu qu'il y a soixante-quatre combinaisons possibles de séquences de trois nucléotides, tandis qu'il n'existe que vingt acides aminés naturels. De ce fait, la plupart des acides aminés sont codés par plusieurs codons. Certains codons dans une espèce donnée sont souvent mieux traduits que les autres codons codant le même acide aminé. En outre, les préférences d'usage de codon varient selon les espèces. De ce fait, il a été observé que l'expression d'un gène d'une espèce peut ne pas être optimale lorsque ce gène est introduit dans une autre espèce. L'homme du métier comprendra aisément qu'il est possible de surmonter ce problème en profitant de la dégénérescence du code génétique. Ainsi, une séquence nucléotidique codant un polypeptide d'intérêt sera modifiée afin que ladite séquence contienne maintenant des codons qui sont utilisés efficacement dans les espèces d'intérêt, mais sans altérer la séquence du polypeptide codé. Il est possible de déterminer quels sont les codons les plus largement utilisés dans l'organisme d'intérêt. Cela a déjà été fait pour un grand nombre d'organismes, y compris les organismes dont dérivent les systèmes acellulaires les plus couramment utilisés (E. coli, lapin, blé). L'homme du métier sera donc pleinement en mesure de déterminer l'usage des codons pour chaque organisme d'intérêt.

Selon un mode de réalisation particulier, ces protéines acellulaires de l'invention sont fusionnées à une séquence d'étiquetage afin d'en faciliter la récupération ultérieure, voire la purification. De telles séquences sont bien connues de l'homme du métier. Elles comprennent les épitopes HA, FLAG, V5 et myc, ainsi que la chitin binding protein (CBP), la maltose binding protein (MBP), la glutathione-S-transférase (GST) et le Strep tag. Il est aussi possible d'utiliser une séquence de 6 histidines. Ces séquences peuvent être ajoutées à l'extrémité N-terminale ou C-terminale de la protéine d'intérêt.

La présente demande décrit mais sans le revendiquer un procédé de synthèse d'une protéine érythrocytaire choisie parmi RhD, RhCE, RhAG et UTB, ou d'un variant de celle-ci, ledit procédé comprenant les étapes suivantes :
a) la mise en contact d'un acide nucléique codant ladite protéine ou le variant de celle-ci avec un système acellulaire de production de protéines, en présence d'au moins un détergent non-ionique, de liposomes ou de nanodisques ; et
b) la synthèse de ladite protéine.

Selon un premier aspect, la présente invention a pour objet un procédé de synthèse d'une protéine érythrocytaire choisie parmi RhD, RhCE, RhAG et UTB, ou d'un variant de celle-ci, ledit procédé comprenant les étapes suivantes :
a) la mise en contact d'un acide nucléique codant ladite protéine ou un homologue ayant au moins 95% d'identité avec celle-ci avec un système acellulaire de production de protéines, en présence d'au moins un détergent non-ionique, de liposomes ou de nanodisques ; et
b) la synthèse de ladite protéine.

Par « système acellulaire de production de protéines », on entend au sens de l'invention un système biochimique permettant la synthèse d'une protéine en l'absence d'une cellule. Le système acellulaire de production de protéines au sens de l'invention contient donc tous les éléments nécessaires pour la production de protéines en l'absence d'une cellule. En particulier, ce système comprend, entre autres, la machinerie transcriptionnelle et traductionnelle provenant de la cellule.

Ces systèmes in vitro sont particulièrement avantageux en ce qu'ils permettent la synthèse de protéines membranaires cytotoxiques ou de protéines régulatrices ou instables, lesquelles ne peuvent pas être exprimées dans des organismes vivants, et donc dans les systèmes in vivo classiques. En particulier, les systèmes acellulaires sont particulièrement adaptés à l'expression de protéines membranaires telles que les protéines érythrocytaires de l'invention. Les protéines membranaires sont en effet très difficiles à exprimer dans des cellules, l'expression à la membrane des cellules nécessitant un trafic intracellulaire et un adressage correct. La plupart des techniques applicables aux protéines solubles ne parviennent pas à faire face aux agrégats insolubles, notamment durant les phases d'extraction et de purification. En revanche, les systèmes acellulaires présentent l'avantage par rapport aux systèmes de synthèse de protéines classiques d'offrir la possibilité de modifier l'environnement de réaction de la protéine de façon assez simple, par exemple avec l'ajout de réactifs favorisant le bon repliement. De fait, chaque paramètre de la réaction (tel que le pH, le potentiel redox, la force ionique,...) peut être modifié en fonction de la protéine cible à produire. En outre, dans ces systèmes, la protéine recombinante résultante représente le produit majeur de la réaction.

La matrice pour la synthèse de protéine sans cellule peut être tout type de polynucléotide, ARN ou ADN. Préférentiellement, la matrice utilisée est une molécule d'ADN. Dans ce cas, le système acellulaire permet de convertir l'information contenue dans la matrice d'ADN en protéine grâce au couplage des réactions de transcription et de traduction.

Le système couplé, notamment utilisé dans les systèmes de E. coli, génère de l'ARNm en continu à partir d'une matrice d'ADN comprenant un promoteur reconnaissable. Dans ce système, une ARN polymérase endogène peut être utilisée, mais il est préférable d'ajouter une ARN polymérase exogène, typiquement celle du phage T7 ou celle du phage SP6, au mélange réactionnel. Le système peut être utilisé avec n'importe quel gène d'intérêt. En particulier, la matrice d'ADN selon l'invention comprend une cassette d'expression permettant d'exprimer la protéine érythrocytaire d'intérêt.

Par « cassette d'expression », on entend ici un fragment d'ADN comprenant un polynucléotide d'intérêt, par exemple un polynucléotide codant une des protéines érythrocytaires de l'invention, lié de façon fonctionnelle à un ou plusieurs éléments régulateurs contrôlant l'expression des séquences géniques, comme, par exemple, les séquences promotrices et les séquences « enhancers ».

Un polynucléotide est « lié de façon fonctionnelle » à des éléments régulateurs quand ces différentes séquences d'acides nucléiques sont associées sur un seul fragment d'acide nucléique de telle façon que la fonction de l'une est affectée par les autres. Par exemple, une séquence d'ADN régulatrice est « liée de façon fonctionnelle » à une séquence d'ADN codant un ARN ou une protéine si les deux séquences sont situées de telle façon que la séquence d'ADN régulatrice affecte l'expression de la séquence d'ADN codante (autrement dit, que la séquence d'ADN codante soit sous le contrôle transcriptionnel du promoteur). Les séquences codantes peuvent être liées de façon fonctionnelle à des séquences régulatrices aussi bien dans une orientation sens que dans une orientation antisens. Préférablement, les séquences codantes de l'invention sont liées de façon fonctionnelle aux séquences régulatrices dans l'orientation sens.

Par « séquences régulatrices » ou « éléments régulateurs », on entend ici des séquences polynucléotidiques qui sont nécessaires pour affecter l'expression et la maturation des séquences codantes auxquelles elles sont ligaturées. De telles séquences régulatrices comprennent notamment les séquences d'initiation et de terminaison de la transcription, les séquences promotrices et les séquences « enhancer » ; les signaux de maturation efficace des ARN, comme les signaux d'épissage et de polyadénylation ; les séquences stabilisant les ARNm cytoplasmiques ; les séquences améliorant l'efficacité de la traduction (par exemple, les séquences de Kozak) ; les séquences qui augmentent la stabilité des protéines ; et, si nécessaire, des séquences qui augmentent la sécrétion des protéines.

De préférence, les séquences régulatrices de l'invention comprennent des séquences promotrices, c'est-à-dire que le gène codant la protéine érythrocytaire de l'invention est de préférence lié de façon fonctionnelle à un promoteur qui permet l'expression de l'ARNm correspondant. Un gène codant une protéine érythrocytaire est de préférence lié de façon fonctionnelle à un promoteur quand il est situé en aval de ce dernier, formant ainsi une cassette d'expression.

On entend ici par « promoteur » une séquence nucléotidique, le plus souvent située en amont (5') de la séquence codante, qui est reconnue par l'ARN polymérase et les autres facteurs nécessaires pour la transcription, et ainsi contrôle l'expression de ladite séquence codante. Un « promoteur » tel qu'on l'entend ici comprend en particulier les promoteurs minimaux, c'est-à-dire de courtes séquences d'ADN composées d'une boite TATA et d'autres séquences qui permettent de spécifier le site de démarrage de la transcription. Un « promoteur » au sens de l'invention comprend aussi des séquences nucléotidiques incluant un promoteur minimal et des éléments régulateurs capables de contrôler l'expression d'une séquence codante. Par exemple, les séquences promotrices de l'invention peuvent contenir des séquences régulatrices comme des séquences « enhancer » pouvant influencer le niveau d'expression d'un gène.

Avantageusement, les promoteurs selon l'invention sont ceux qui fonctionnent avec l'ARN polymérase utilisée dans le système acellulaire d'intérêt. Par exemple, des promoteurs reconnus par les ARN polymérases des phages SP6 et T7 sont largement connus de l'homme du métier. Ainsi les vecteurs pIVEX qui portent des promoteurs reconnus par l'ARN polymérase T7 (Rogé and Betton, 2005) ont été utilisés dans la partie expérimentale ci-après. Des vecteurs contenant de tels promoteurs sont par ailleurs disponibles commercialement.

Selon l'invention, des détergents, des liposomes ou des nanodisques sont ajoutés dans le milieu réactionnel, soit au cours de la synthèse protéique, soit de manière préférentielle avant même que celle-ci ne débute. Cet ajout lipidique se fait préférentiellement à hauteur de quelques milligrammes par ml de milieu réactionnel, généralement entre 0,5 et 10 mg/ml.

Le terme «détergent» tel qu'utilisé ici signifie une molécule amphipathique contenant à la fois des groupes hydrophobes et hydrophiles. Ces molécules contiennent un groupe hydrophile polaire et une longue chaîne de carbone hydrophobe. On entend par «détergent non ionique» une molécule qui contient un détergent dont le groupement hydrophile est non chargé. Les détergents non-ioniques au sens de l'invention comprennent entre autres les alkyle polyglucosides, l'octaethylene glycol monododecyl ether (C12E8), les détergents de la famille du Brij tels que, par exemple, le Brij 35 (C12E23Polyoxyethyleneglycol dodecyl ether) ou le Brij 58 (C16E20 Polyoxyethyleneglycol dodecyl ether), le Genapol, les glucanids comme le MEGA-8, - 9, -10, octylglucoside, le Pluronic F127, les détergents de la famille du Triton tels que le Triton X-100 (C14H22O(C2H4O)n) ou le Triton X-114 (C24H42O6), et les détergents de la famille du Tween, notamment le Tween 20 (Polysorbate 20) et le Tween 80 (Polysorbate 80). De façon préférée, le détergent non-ionique de l'invention est choisi parmi le Brij 35 et le Brij 58. De façon plus préférée, le détergent non-ionique de l'invention est le Brij 35.

Selon ce mode de réalisation préféré, l'invention a donc pour objet un procédé comme décrit ci-dessus, dans lequel la mise en contact selon l'étape a) se fait en présence d'au moins un détergent non-ionique sélectionné parmi le Brij 35 ou le Brij 58.

Les concentrations les plus efficaces de détergent pour produire les protéines érythrocytaires selon la méthode de l'invention varient d'un détergent à l'autre. Elles varient en fonction de la basse concentration micellaire critique du détergent, c'est-à-dire la concentration à partir de laquelle des micelles se forment. Néanmoins, ces concentrations sont typiquement comprises entre 0,1 et 5 %, plus particulièrement entre 0,1 et 1 %. Par exemple, le Brij 35 et le Brij 58 sont typiquement utilisés à 0,5 %. Selon que le détergent considéré soit solide ou liquide, % se réfère respectivement à un rapport % m/v ou % v/v.

Par « liposome », on entend ici une vésicule artificielle formée par des bicouches lipidiques concentriques, emprisonnant entre elles des compartiments aqueux. Les liposomes peuvent être constitués de n'importe quel lipide convenable, y compris, mais sans s'y limiter, les lipides polaires tels que des phospholipides, tels que les phosphoglycérides, tels que la phosphatidyléthanolamine, la phophatidylcholine, la phosphatidylsérine, la cardiolipine, ou leurs combinaisons. D'autres composés lipidiques peuvent également être incorporés dans les liposomes tels que les triacylglycérols, des cires, des sphingolipides, et des stérols et leurs esters d'acides gras, ou des combinaisons de ceux-ci.

Les vésicules lipidiques correspondent à des bicouches lipidiques se présentant sous forme de sphères d'un diamètre d'environ 100 nm, préparées à l'aide de protocoles connus de l'homme du métier.

Ces vésicules lipidiques peuvent être traitées à l'aide de détergents, avant leur introduction dans le milieu réactionnel. Selon un premier mode de réalisation, les vésicules lipidiques mises en œuvre sont d'origine naturelle, préférentiellement issues de soja ou d'oeufs. De tels lipides sont disponibles commercialement chez Avanti Polar Lipids revendus par Coger en France. Alternativement, les vésicules lipidiques peuvent être des liposomes d'origine synthétique, c'est à dire produits à partir de lipides synthétiques.

Selon un mode de réalisation privilégié et en particulier dans le cas de lipides synthétiques, les liposomes mis en œuvre dans le cadre de l'invention sont porteurs de molécules de polyéthylène glycol (PEG), ou de dérivés de PEG (PEG fonctionnalisé) tels que le N-carbonyl-méthoxy-polyéthylène glycol 2000.

Le terme « nanodisque » tel qu'utilisé ici se réfère à au moins une bicouche lipidique stabilisée par une armature protéique. Préférentiellement, cette armature entoure la bicouche lipidique de façon à former une structure discoïde.

Par "lipides", on entend ici toute molécule naturelle liposoluble (c'est-à-dire lipophile). Les lipides sont un groupe hétérogène de composés possédant de nombreuses fonctions biologiques essentielles. Ces composés intervenant ainsi aussi bien en tant que composants structuraux des membranes cellulaires, que sources de stockage de l'énergie ou que molécules intermédiaires dans les voies de signalisation. Les lipides peuvent être définis comme des petites molécules hydrophobes ou amphiphiles, qui proviennent entièrement ou en partie de groupes cétoacyl ou isoprènes. Pour un aperçu d'ensemble de toutes les classes de lipides, on se reportera avantageusement à « Lipid Metabolites and Pathways Strategy (LIPID MAPS) classification system » (National Institute of General Medical Sciences, Bethesda, MD). Les lipides peuvent former des micelles, des membranes monocouches et des membranes bicouches. Les lipides peuvent s'auto-assembler en combinaison le cas échéant avec d'autres composants pour former des nanodisques. Les lipides au sens de l'invention incluent les graisses, les cires, les phospholipides, les sphingolipides (tels que la sphingomyéline), les stérols (tels que le cholestérol), les cérébrosides et les composés qui dérivent de chacun de ces groupes de lipides. Les lipides utilisés dans les nanodisques au sens de l'invention sont de préférence des phospholipides. Un « phospholipide » au sens de l'invention est un lipide contenant un groupe acide phosphorique en tant que mono ou di-ester. Les phospholipides qui peuvent être utilisés dans les nanodisques de l'invention comprennent les phospholipides synthétiques, naturels, saturés, insaturés, leurs dérivés (par exemple, acyl, diéther et lyso) et toute combinaison de phospholipides de classes différentes ou identiques pouvant favoriser une bonne insertion et conformation de la protéine exprimée et, par là même, sa reconnaissance par des ligands ou des anticorps. Les phospholipides comprennent entre autres la phosphatidylcholine, l'acide phosphatidique, la phosphatidyléthanolamine, le phosphatidylglycérol, la phosphatidylsérine, le phosphatidylinositol,le phosphatidylinositolphosphate, la cardiolipine et leurs dérivés. Dans un mode de réalisation préféré, les phospholipides comprennent les dioleoyl, dimyristoyl, palmitoyl-oléoyl glycéro-phosphocholines (DOPC, DMPC, POPC), les palmitoyl-oléoyl, dimyristoyl et dioleoyl phosphoglycérol (POPG, DMPG et DOPG). Dans un mode de réalisation encore plus préféré, les phospholipides comprennent les dioleoyl, dimyristoyl, palmitoyl-oléoyl glycéro-phosphocholines (DOPC, DMPC, POPC). Selon un mode de réalisation particulièrement avantageux, les phospholipides de l'invention sont combinés avec le cholate de sodium. Selon ce mode de réalisation, l'élimination de ce sel, par exemple par dialyse, permet d'obtenir des nanodisques à partir des phospholipides et de l'armature protéique de l'invention.

Le terme « armature protéique » tel qu'il est ici utilisé englobe toute protéine capable de s'assembler avec un lipide amphipathique dans un environnement aqueux et d'organiser ledit lipide amphipathique en une bicouche. Préférentiellement, l'armature protéique de la présente invention doit être amphipathique, avec une partie de sa structure plus ou moins hydrophile et en regard du solvant aqueux et l'autre partie plus ou moins hydrophobe et faisant face au centre de la bicouche hydrophobe qui est ainsi stabilisée. Le terme « armature protéique » inclut ainsi, sans toutefois y être limité, les apolipoprotéines, les lipophorines, leurs dérivés (comme, par exemple, des séquences tronquées et organisées en tandem) et leurs fragments (c'est-à-dire des peptides), comme l'apolipoprotéine E4, le fragment 22K, la liphorine III, l'apolipoprotéine A-I et les molécules similaires. L'armature protéique peut également être constituée par des peptides amphipathiques artificiels conçus spécifiquement dans ce but. Dans certains modes de réalisation particuliers, ces peptides sont des peptides amphipathiques hélicoïdes qui miment les hélices alpha d'une apolipoprotéine qui sont orientées perpendiculairement aux chaines d'acides gras des lipides amphipathiques, notamment celles des phospholipides. De tels peptides sont décrits dans la demande WO 2008/141230 A1.

De préférence, l'armature protéique des nanodisques de l'invention est constituée par la protéine MSP1, ou un dérivé de celle-ci. MSP1 est une forme tronquée de l'apoliprotéine A-I, qui présente la même structure amphipathique hélicoïde que la protéine complète. La protéine MSP1 possède la séquence : GLKLLSNWDSVTSTFSKLREQLGPVTQEFWDNLEKETEGLRQEMSKDLEEVKAKVQPYLDDFQKKW QEEMELYRQKVEPLRAELQEGARQKLHELQEKLSPLGEEMRDRARAHVDALRTHLAPYSDELRQRL AARLEALKENGGARLAEYHAKATEHLSTLSEKAKPALEDLRQGLLPVLESFKVSFLSALEEYTKKLNT Q (SEQ ID NO. 9).

Des dérivés de cette protéine ont été construits (voir par exemple, Grinkova et al., 2010, WO 02/40501, WO 2005/081743, US 7,083,958 B2). Certains de ces dérivés comprennent des séquences permettant la purification facile de la protéine et des nanodisques dans lesquels elle est incorporée. Ainsi une séquence d'étiquetage peut être ajoutée à la protéine. De telles séquences sont bien connues de l'homme du métier. Elles comprennent les épitopes HA, FLAG, V5 ou myc, ainsi que la chitin binding protein (CBP), la maltose binding protein (MBP), la glutathione-S-transférase (GST) et le Strep tag, ou une séquence de 6 histidines. Par exemple, une séquence de 6 histidines peut être fusionnée en N-terminal de la protéine MSP1. Certains dérivés de MSP1 contiennent un site reconnu par la protéase TEV, qui a été modifié de façon à conduire à un clivage complet et spécifique. Les dérivés de MSP1 peuvent aussi comprendre une troncature supplémentaire Δ(1-11) en N-terminal de MSP1. Cette troncature donne des disques plus stables, l'intégralité de la première hélice n'étant pas nécessaire pour l'interaction avec les lipides. La protéine M1PD1 (SEQ ID NO. 10) comprend ainsi la troncature Δ(1-11) fusionnée en N-terminal à une séquence His6 et à un site TEV. La protéine MSP1D1 génère des nanodisques d'un diamètre d'environ 9,7 nm qui contiennent typiquement entre 120 et 160 molécules de lipides et deux MSPs par nanodisque. Un autre dérivé couramment utilisé par l'homme du métier est la protéine MSP1E3D1 (SEQ ID NO. 11) qui comporte une insertion de trois hélices additionnelles (hélices 4, 5 et 6) entre les hélices 3 et 4 de la protéine MSP1D1. Cette protéine comprend donc une duplication des hélices 4, 5 et 6 de la protéine MSP1D1. De ce fait, elle génère des nanodisques qui ont une taille plus importante que ceux obtenus avec la protéine MSP1D1 : environ 12,9 nm. Ces deux dérivés, MSP1D1 et MSP1E3D1, sont les plus couramment utilisés pour générer des nanodisques. Ils sont d'ailleurs tous deux disponibles commercialement (chez Sigma-Aldrich ou Cube Biotech entre autres). Très préférentiellement, la protéine MSP de l'invention est une protéine ayant une séquence choisie parmi SEQ ID NO. 9, 10 ou 11.

Selon ce mode de réalisation préféré, l'invention a pour objet un procédé tel que décrit ci-dessus, dans lequel la mise en contact selon l'étape a) se fait en présence de nanodisques comprenant une protéine MSP choisie parmi les protéines de SEQ ID NO. 9, 10 ou 11.

L'invention a donc pour objet un procédé tel que décrit ci-dessus, dans lequel la mise en contact selon l'étape a) se fait en présence d'au moins un détergent non-ionique sélectionné parmi le Brij 35 ou le Brij 58, ou de nanodisques comprenant une protéine MSP choisie parmi les protéines de SEQ ID NO. 9, 10 ou 11.

Les inventeurs ont montré que l'utilisation de nanodisques permettait de produire les protéines érythrocytaires de l'invention à l'état soluble et en grande quantité. En particulier, ils ont montré qu'il est important pour optimiser la production d'utiliser une concentration en nanodisques adéquate. A cet égard, une concentration en nanodisques inférieure à 80 µM est particulièrement adaptée pour obtenir une grande quantité de protéine à l'état soluble. Par exemple, des concentrations en nanodisques de 20 µM, 40 µM ou 60 µM permettent d'obtenir des rendements au moins aussi bons que ceux obtenus en présence de détergent.

Les méthodes pour générer des nanodisques avec des lipides, préférentiellement des phospholipides, et une protéine qui peut former une armature sont bien connues de l'homme du métier (voir par exemple Denisov et al., 2004). On ne les détaillera donc pas ici, mais on se bornera à noter qu'en outre de nombreuses sociétés proposent des nanodisques (par exemple, Cube Biotech, Sigma Aldrich, Nanodiscs Inc.).

Le système acellulaire a comme principe de base l'utilisation de la machinerie transcriptionnelle et traductionnelle d'un organisme afin de produire à partir d'une information génétique exogène une protéine recombinante spécifique. Les organismes à partir desquels cette machinerie est extraite sont multiples et variés, et proviennent d'organismes procaryotes et eucaryotes.

De tels systèmes sont bien connus de l'homme du métier depuis plusieurs dizaines d'années (pour une revue, voir par exemple : Carlson et al., 2012). De nombreuses méthodes sont disponibles pour synthétiser des protéines dans des systèmes acellulaires (voir par exemple Cell-free protein synthesis: Methods and protocols, edited by Alexander S. Spirin and James R. Swartz. 2008. Wiley-VCH, Weinheim, Germany). Il est aussi possible d'utiliser des kits proposés par de nombreuses sociétés : Qiagen, Ambion, Promega, Invitrogen, Thermo Scientific, Roche Diagnostics, CellFree Sciences & Co, etc.

De façon préférée, les systèmes acellulaires de production de protéines de l'invention comprennent des extraits cellulaires. Bien que n'importe quel organisme puisse être utilisé comme source d'extraits cellulaires, il est préférable d'utiliser des extraits de la bactérie Escherichia coli, de réticulocytes de lapin, de germes de blé ou de cellules d'insectes. Les extraits d'E. coli sont particulièrement avantageux. Tout d'abord, l'homme du métier dispose déjà d'une longue expérience de ce système, qui est de loin le plus populaire. Ces extraits peuvent être préparés sans difficulté et à un coût peu élevé. Ils permettent d'obtenir des rendements très élevés pour un coût énergétique plus faible que les autres systèmes. Ce système est aisément disponible commercialement, puisqu'actuellement plusieurs entreprises commercialisent des kits d'expression acellulaire de protéines utilisant des extraits d'E. coli : Qiagen, Promega, Invitrogen, Thermo Scientific, Roche diagnostics, etc.

Il existe deux façons d'effectuer la réaction sans cellule. Dans les réactions discontinues, aussi appelées réactions en batch, la transcription et la traduction sont effectuées dans un volume de réaction contenant tous les composants nécessaires. Pour différentes raisons, comme l'épuisement rapide de l'approvisionnement énergétique, la dégradation des composants tels que des nucléotides et des concentrations décroissantes des ions Mg²⁺ libres, la réaction dans le système en batch atteint généralement un plateau après environ 1-2 heures. L'utilisation d'un système optimisé d'expression in vitro en batch produit généralement jusqu'à 500 µg de protéines/ml, même si des valeurs plus élevées peuvent parfois être atteintes au prix d'optimisations supplémentaires.

Dans un mode de dialyse, la réaction de transcription / traduction sans cellules est effectuée dans une petite chambre de réaction qui est séparée par une membrane de dialyse (généralement de 10 à 15 kDa de coupure) d'un réservoir environ 10 à 20 fois plus grand contenant des réactifs de bas poids moléculaire.

Dans le premier des systèmes en mode dialyse, le système de production acellulaire en flux continu ou Continuous Flow Cell Free (CFCF), le milieu réactionnel est séquestré par une membrane d'ultrafiltration et est continuellement alimenté par une pompe. Cette membrane permet de laisser passer la protéine d'intérêt dans le compartiment de tampon et ainsi de la récupérer tout en continuant d'alimenter la réaction et ainsi pouvoir faire des réactions durant plusieurs dizaines d'heures.

Dans un système de production acellulaire en échange continu (Continuous Exchange Cell Free ou CECF), le compartiment de réaction contenant le lysat cellulaire et l'information génétique est séparé par une membrane de dialyse d'un compartiment de nutrition contenant les cofacteurs, acides aminés, tampons et d'autres composants. La membrane de dialyse permet la sortie des molécules déchets, mais laisse rentrer les composants permettant à la réaction de s'effectuer grâce au gradient de concentration résultant de l'activité de synthèse. Cette configuration permet d'augmenter significativement la durée de réaction et les niveaux de protéine produite. Des rendements allant jusqu'à 5 mg / ml dans le volume de réaction ont été rapportées pour le système de E. coli avec ce procédé, à la fois avec un système commercial (RTS, Roche Applied Science) et les systèmes faits maison.

Le système acellulaire de l'invention est un système en batch ou en mode de dialyse. Préférentiellement, ce système est un système mode de dialyse. Plus préférentiellement, ce système est un système de production acellulaire en échange continu.

L'avantage du système acellulaire est qu'il offre la possibilité de contrôler précisément les paramètres de la réaction. Outre les extraits, les systèmes acellulaires de production de protéines comprennent plusieurs éléments dont la concentration peut être critique pour l'efficacité de la réaction.

Les ions divalents Mg²⁺ sont essentiels dans un grand nombre de réactions biologiques. En l'espèce, les inventeurs ont montré qu'une concentration de magnésium comprise entre 14 et 22 mM, de préférence comprise entre 16 et 20 mM permet d'obtenir des rendements appréciables de protéines érythrocytaires. Le système acellulaire de production de protéines selon l'invention comprend ainsi une concentration de magnésium comprise entre 14 et 22 mM, de préférence comprise entre 16 et 20 mM.

D'autres sels, en particulier ceux qui sont biologiquement pertinents, tels que le manganèse, peuvent aussi être ajoutés. Le potassium est généralement présent à une concentration d'au moins environ 50 mM, et pas plus d'environ 250 mM. L'ammonium peut être présent, habituellement à une concentration inférieure à 200 mM, plus généralement à une concentration inférieure à environ 100 mM. Habituellement, la réaction est maintenue dans l'intervalle de pH d'environ 5 à 10 et une température d'environ 20° -50° C ; plus généralement, dans la gamme de pH d'environ 6-9 et une température d'environ 25° -40° C. Avantageusement, la réaction est mise en œuvre à pH = 7,5. Par ailleurs, une température de 20°C est particulièrement avantageuse pour cette réaction. Ces gammes peuvent être étendues pour des conditions spécifiques d'intérêt.

En revanche, il est inutile d'ajouter des co-facteurs exogènes pour l'activation de la phosphorylation oxydative. Des composés tels que le nicotinamide-adénine-dinucléotide (NADH), le NAD⁺, ou l'acétyl-coenzyme A peuvent être utilisés pour compléter les rendements de synthèse de protéines mais ne sont pas nécessaires. L'addition d'acide oxalique, un inhibiteur du métabolisme du phosphoénolpyruvate synthétase (Pps), peut être bénéfique en augmentant les rendements de protéines, mais n'est pas nécessaire.

Les protéines érythrocytaires susceptibles d'être obtenues selon le procédé de l'invention sont des protéines membranaires. Or, il est important que les protéines membranaires produites dans le système acellulaire soient correctement repliées et fonctionnelles. En particulier, il est important d'éviter la formation d'agrégats. A cet égard, les inventeurs ont montré qu'une durée de réaction comprise entre 2 et 24 heures, de préférence entre 6 et 12 heures, plus préférentiellement d'environ 8 heures, permet de maximiser le rendement tout en évitant les agrégats.

Selon un mode de réalisation particulier, le procédé de l'invention comprend une étape de récupération de la protéine produite. Cette étape peut être facilitée par l'utilisation d'une séquence d'étiquetage. Selon un mode de réalisation particulier, cette séquence est fusionnée à la protéine érythrocytaire d'intérêt. Il est à noter que, lorsque la protéine érythrocytaire susceptible d'être obtenue selon le procédé de l'invention est produite en présence de nanodisques, il peut être avantageux de fusionner la protéine composant les nanodisques à une séquence d'étiquetage et de s'en servir pour récupérer la protéine de l'invention.

L'isolement (ou la purification) de la protéine érythrocytaire susceptible d'être obtenue selon le procédé de l'invention peut se faire par tout moyen connu de l'homme du métier. On citera par exemple la précipitation différentielle ou l'ultracentrifugation. Il peut être également avantageux de purifier les fragments d'intérêt par chromatographie d'échange ionique, par chromatographie d'affinité, par tamisage moléculaire, ou par isofocalisation. Toutes ces techniques sont décrites dans Voet D et Voet JG, Techniques de purification des protéines et des acides nucléiques, chapitre 6, Biochimie, 2nde édition. Préférentiellement, la protéine d'intérêt peut être récupérée par immunoprécipitation en utilisant par exemple des anticorps dirigés contre cette protéine. Alternativement, la protéine d'intérêt peut, le cas échéant, être isolée par chromatographie d'affinité à l'aide de la séquence d'étiquetage.

Il peut être particulièrement utile dans certaines applications de coupler la protéine érythrocytaire d'intérêt à un support solide : par exemple, pour détecter dans le sérum d'un sujet des anticorps dirigés contre des allo-antigènes portés par la protéine. Le couplage de ladite protéine peut être direct ou indirect. Le couplage de la protéine est direct quand la protéine interagit avec le support solide sans l'intermédiaire d'une autre protéine. C'est le cas par exemple quand le couplage est réalisé via des anticorps dirigés contre la protéine d'intérêt. C'est aussi le cas, quand la protéine d'intérêt est elle-même fusionnée à une séquence d'étiquetage. Un couplage indirect au sens de l'invention signifie un couplage médié par une autre protéine, par exemple par la MSP ou par une fusion entre une protéine MSP et une séquence d'étiquetage. Dans ce cas, ladite autre protéine est couplée au support solide et la protéine d'intérêt n'est elle-même couplée à celui-ci que dans la mesure où elle interagit avec ladite autre protéine. Ainsi, par exemple, la protéine d'intérêt est couplée de façon indirecte à un support solide quand elle est exprimée dans des nanodisques dont la protéine MSP est liée audit support solide. Ce mode de couplage peut être avantageux pour ne pas perturber l'organisation tridimensionnelle de la protéine d'intérêt.

Selon ce mode particulier de réalisation, le procédé de l'invention comprend une étape supplémentaire de fixation de la protéine érythrocytaire, ou son variant, et/ou de la protéine MSP à un support solide. Avantageusement, sur ledit support solide est greffé un composé qui interagit spécifiquement avec la protéine érythrocytaire, ou son variant, et/ou la protéine MSP. Ledit composé est, par exemple, un anticorps dirigé contre la protéine érythrocytaire d'intérêt ou contre la protéine MSP. Préférentiellement, ce composé est reconnu par la séquence d'étiquetage présente dans la protéine érythrocytaire, ou son variant, et/ou la protéine MSP. Ce composé peut être un anticorps reconnaissant un épitope spécifique, comme les épitopes HA, FLAG, V5 ou myc. Il peut aussi être un sucre (la chitine ou le maltose, par exemple) ou un métabolite (comme le glutathion) qui est lié par une protéine (CBP, MBP et GST, respectivement). Ledit composé peut aussi être un peptide, éventuellement modifié par génie génétique, qui est reconnu et lié par une séquence peptidique spécifique : ainsi, le peptide Strep-Tactin est dérivé par modifications génétiques de la streptavidine et est lié par une séquence spécifique, le Strep-tag. Enfin, ce composé peut être un ion divalent comme Ni²⁺, qui est lié par une séquence de 6 histidines. Les méthodes pour coupler ces composés à des supports solides sont bien connues de l'homme du métier. On ne les détaillera donc pas ici.

Le support solide qui peut être utilisé dans la présente invention n'est limité en aucune façon, tant que c'est un support solide ou réalisé en un matériau insoluble (par exemple, un matériau qui peut être séparé d'un mélange réactionnel par filtration, précipitation, séparation magnétique ou toute autre technique appropriée).

Les matériaux qui constituent ledit support solide comprennent, sans y être limités, la cellulose, le Téflon ^{™}, la nitrocellulose, l'agarose, le dextrane, le chitosane, le polystyrène, le polyacrylamide, le polyester, le polycarbonate, le polyamide, le polypropylène, le nylon, polydivinylidene difluorure, de latex, de la silice , le verre, la fibre de verre, l'or, le platine, l'argent, le cuivre, le fer, l'acier inoxydable, la ferrite, plaquette de silicium, le polyéthylène, la polyéthylèneimine, l'acide polylactique, les résines, les polysaccharides, les protéines (par exemple l'albumine), le carbone et les combinaisons de ceux-ci.

Le support solide peut avoir n'importe quelle forme, y compris, mais sans s'y limiter, celle de bille, de bille magnétique, de film mince, de microtube, de filtre, de plaque, de microplaque, de nanotube de carbone, de puce de capteur, etc. Les supports solides plats tels que des films ou des plaques minces peuvent aussi comprendre des puits, des canaux, des fonds de filtre ou autres, comme cela est connu dans l'art. En fait, le support solide peut être toute surface sur laquelle le composé reconnu par la séquence d'étiquetage peut se lier. Le support solide selon l'invention comprend, entre autres, les plaques de microtitration, des billes, des disques, des puces, des diapositives et tout autre support convenable.

Dans un mode de réalisation de la présente invention, le support solide est constitué par des billes magnétiques ayant un diamètre sphérique compris entre environ 25 nm et environ 1 mm. Dans un mode de réalisation préféré, les billes magnétiques ont un diamètre compris entre environ 50 nm et environ 10 µm. La taille des billes magnétiques peut être choisie en fonction de l'usage prévu.

Selon un autre mode de réalisation de la présente invention, le support solide est constitué par des billes composées d'agarose sphérique hautement réticulé (par exemple, la sépharose). Préférablement, lesdites billes ont un diamètre compris entre environ 24 µm et environ 165 µm. Dans un mode de réalisation plus préféré, lesdites billes ont un diamètre compris entre environ 24 µm et environ 44 µm. La taille de ces billes d'agarose sphérique hautement réticulé peut être choisie en fonction de l'usage envisagé.

Les supports solides ayant une surface hydrophobe comprennent entre autres des billes de latex de polystyrène, telles que celles disponibles dans le commerce auprès de Polysciences, Warrington, PA ou Spherotech, Liberville, IL.

Des exemples de silice (SiO₂) imprégnées ou de la silice (SiO₂) à base de support solide comprennent des billes de silice superparamagnétiques qui sont disponibles auprès de Polysciences, Warrington, PA. Il est également possible d'utiliser M-280 disponible dans le commerce auprès de Dynal Biotech, etc.

Les billes magnétiques ayant une surface hydrophile peuvent être utilisées dans la méthode de la présente invention. Des exemples de ces billes magnétiques incluent les billes disponibles dans le commerce sous le nom Biomag^{®} carboxyle auprès de Polysciences, Warrington, PA ou de billes MC02N Nom de famille / 2928 de Bangs Laboratory, Inc., Fishers, IN. Il est également possible d'utiliser M-270 disponible dans le commerce auprès de Dynal Biotech, etc.

Est également décrite ici une protéine érythrocytaire exprimée dans un nanodisque, un liposome ou un détergent non-ionique, ou un homologue ayant au moins 95% d'identité avec celle-ci, ladite protéine ou son variant étant susceptible d'être obtenue par le procédé ci-dessus.

La protéine érythrocytaire est sélectionnée parmi RhD, RhCE, RhAG et UTB, ou d'un variant de celle-ci, ladite protéine ou son variant étant susceptible d'être obtenue par le procédé ci-dessus, et est couplée à un support solide. Avantageusement, la protéine érythrocytaire selon l'invention est sélectionnée parmi RhD, RhCE, RhAG et UTB, ou un homologue ayant au moins 95% d'identité avec celle-ci, ladite protéine ou son homologue étant susceptible d'être obtenue par le procédé selon l'invention, et est couplée à un support solide. La protéine érythrocytaire ainsi obtenue est correctement repliée, cela contrairement à l'art antérieur extrayant des protéines à partir de globules rouges sans conserver la conformation. Les inventeurs ont en effet montré que la protéine RhD produite par le procédé de l'invention est reconnue par des anticorps conformationnels utilisés habituellement pour caractériser la protéine endogène native. Avantageusement, la protéine érythrocytaire obtenue par le procédé de l'invention est fonctionnelle.

En outre, est décrite ici une composition comprenant une protéine érythrocytaire choisie parmi RhD, RhCE, RhAG et UTB, ou un variant de celle-ci. De préférence, la concentration de la protéine contenue dans cette composition est supérieure à 0,01 mg/ml, plus préférablement supérieure à 0,1 mg/ml, encore plus préférablement supérieure à 0,5 mg/ml et toujours plus préférablement supérieure à 1mg/ml.

L'utilisation de liposomes ou de nanodisques dans le procédé de l'invention génère des particules, protéoliposomes ou nanodisques de lipides et de protéines, qui comprennent à la fois la protéine érythrocytaire d'intérêt et des lipides. L'invention a donc pour objet, dans un autre aspect, une composition comprenant une protéine érythrocytaire choisie parmi RhD, RhCE, RhAG et UTB, ou un variant de celle-ci, et un ou plusieurs lipides. De préférence, la concentration de la protéine contenue dans cette composition est supérieure à 0,01 mg/ml, plus préférablement supérieure à 0,1 mg/ml, encore plus préférablement supérieure à 0,5 mg/ml et toujours plus préférablement supérieure à 1mg/ml.

Selon encore un autre aspect, l'invention porte sur une composition comprenant une protéine érythrocytaire choisie parmi RhD, RhCE, RhAG et UTB, ou un variant de celle-ci, et une protéine MSP choisie parmi les protéines de SEQ ID NO. 9, 10 ou 11. Une telle composition est notamment obtenue quand des nanodisques sont utilisés dans le procédé de l'invention. Préférablement, la composition de l'invention comprend aussi des lipides. De préférence, la concentration de la protéine érythrocytaire contenue dans une telle composition est supérieure à 0,01 mg/ml, plus préférablement supérieure à 0,1 mg/ml, encore plus préférablement supérieure à 0,5 mg/ml et toujours plus préférablement supérieure à 1mg/ml.

Une protéine érythrocytaire choisie parmi RhD, RhCE, RhAG et UTB, ou un variant de celle-ci, caractérisée en ce que ladite protéine est couplée à un support solide est également décrite ici. Le couplage de ladite protéine peut être direct ou indirect, comme expliqué plus haut.

Les protéines susceptibles d'être obtenues selon le procédé de l'invention sont particulièrement importantes parce qu'elles peuvent être utilisées simplement dans un test de détection d'allo-anticorps antiérythrocytaires. Ce sont des anticorps dirigés contre des antigènes présents à la surface des érythrocytes et qui peuvent induire l'hémolyse de ces derniers. Les allo-anticorps antiérythrocytaires sont produits contre des antigènes érythrocytaires étrangers. L'immunisation a lieu par exemple lors d'une transfusion sanguine, pendant la grossesse ou à la naissance. Si de tels anticorps de type IgG passent la barrière placentaire, ils peuvent induire une destruction accélérée des érythrocytes de l'enfant ou un blocage de l'érythropoïèse fœtale.

Selon ce nouvel aspect de l'invention, celle-ci a pour objet l'utilisation d'une protéine érythrocytaire formulée sous forme de protéoliposome ou d'une composition comprenant une telle protéine érythrocytaire dans un test de détection d'allo-anticorps.

La présence de tels allo-anticorps dans un échantillon biologique d'un sujet comprend notamment la mise en contact dudit échantillon avec une protéine érythrocytaire ou une composition comprenant une protéine érythrocytaire comme décrite ci-dessus, suivie de la détection, le cas échéant, de l'interaction entre la protéine érythrocytaire et des anticorps dirigés contre celle-ci.

Par « sujet » on entend ici un mammifère, de préférence une personne comme, par exemple, une femme enceinte ou un patient transfusé. Tel qu'il est utilisé ici, le terme «échantillon biologique» ou «échantillon» fait référence à un organisme entier ou un sous-ensemble de ses tissus, cellules ou parties de composants. En outre «échantillon biologique» se rapporte à un homogénat, lysat ou extrait préparé à partir d'un organisme entier ou un sous-ensemble de ses tissus, cellules ou composants, ou une fraction ou une partie de celui-ci. De préférence, un "échantillon biologique" selon l'invention est tout tissu qui peut contenir des allo-anticorps. Par « échantillon biologique », on entend ainsi par exemple les échantillons humoraux tels que le sang, le fluide de moelle osseuse, et le liquide lymphatique, et des échantillons solides tels que les ganglions lymphatiques, les vaisseaux sanguins, la moelle osseuse, le cerveau, la rate et de la peau. Plus préférablement, l'échantillon biologique de l'invention est un échantillon de sang, de plasma, ou de moelle osseuse.

L'interaction entre la protéine érythrocytaire d'intérêt et les allo-anticorps correspondants est détectée par tout moyen connu de l'homme du métier. Celui-ci peut en particulier utiliser des technologies bien connues telles que l'immunoprécipitation, l'immunohistologie, le western blot, le dot blot, l'ELISA ou l'ELISPOT, les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérométrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

L'invention sera décrite plus précisément au moyen des exemples ci-dessous.

### LEGENDES DES FIGURES

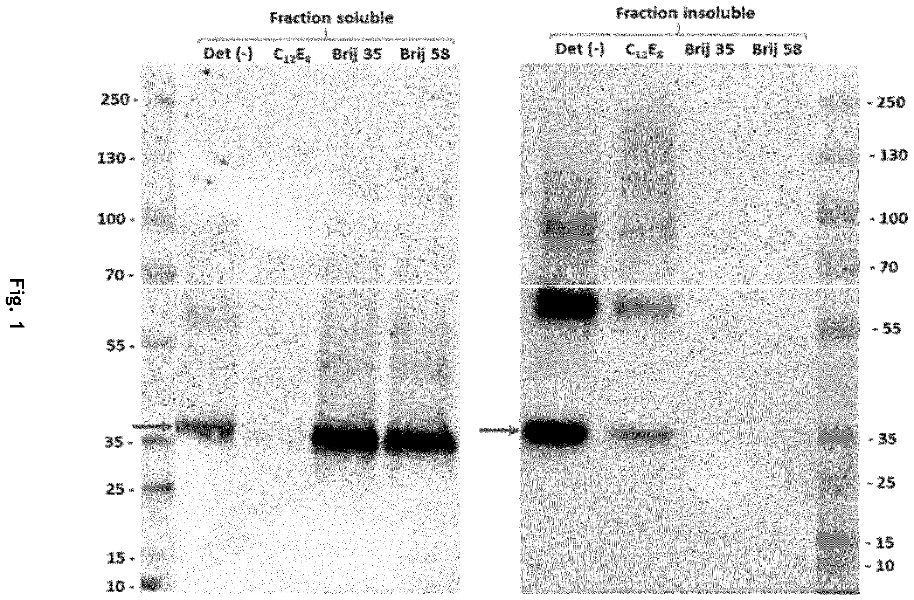

**Figure 1****.** Détection de la protéine RHD par Western-Blot. Les protéines totales des différentes synthèses ont été séparées par centrifugation en fraction soluble et fraction insoluble, la protéine RHD a été révélée par l'anticorps LOR15C9 et un anticorps secondaire anti-humain conjugué à l'HRP (1/1 000). Les marqueurs de poids moléculaires (kDa) sont indiqués à gauche et à droite.

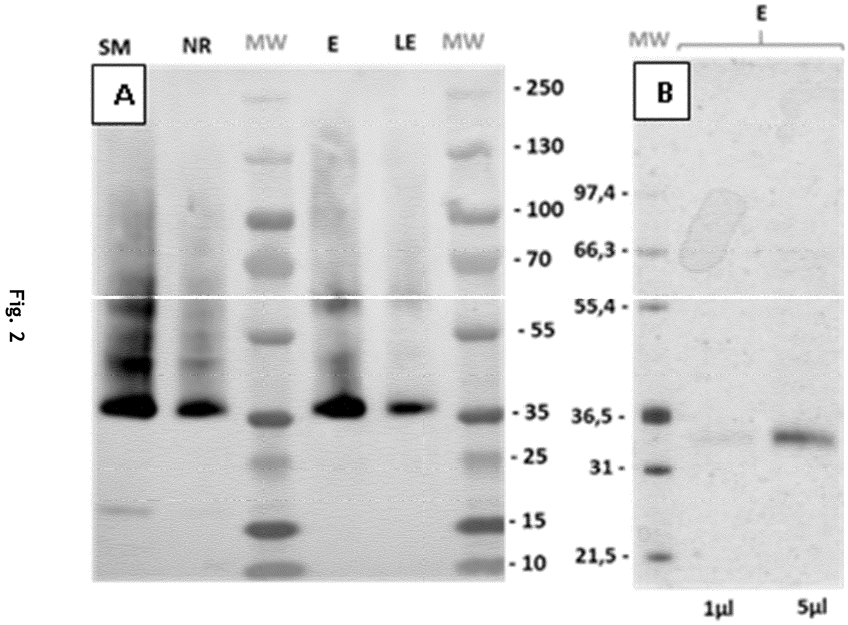

**Figure 2****.** Purification sur Agarose HA de la protéine produite en présence de Brij 35 0.5%. Analyse par western blot des étapes de purification à l'aide d'un anticorps anti-HA (1/2 000) (A) et analyse de l'éluat par coloration au nitrate d'argent (B). La fraction SM correspond au produit de synthèse in vitro de la protéine RHD, NR : Fraction non retenue par la résine, E : Eluat, LE : Lavage d'élution, et MW : marqueur de poids moléculaire (kDa).

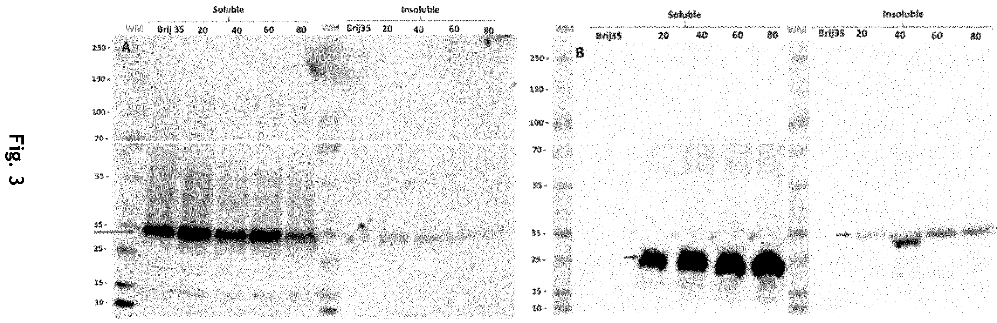

**Figure 3**. Détection des protéines RHD et MSP par Western-Blot. Analyse des produits de synthèse de la protéine RHD en présence de nanodisques ou de Brij 35. (A) La protéine RHD a été révélée par l'anticorps LOR 15C9, et un anticorps secondaire anti-humain couplé à l'HRP (1/1 000). (B) La protéine MSP a été révélée par un anticorps anti-His couplé à l'HRP (1/10 000). MW correspond au marqueur de poids moléculaire (kDa).

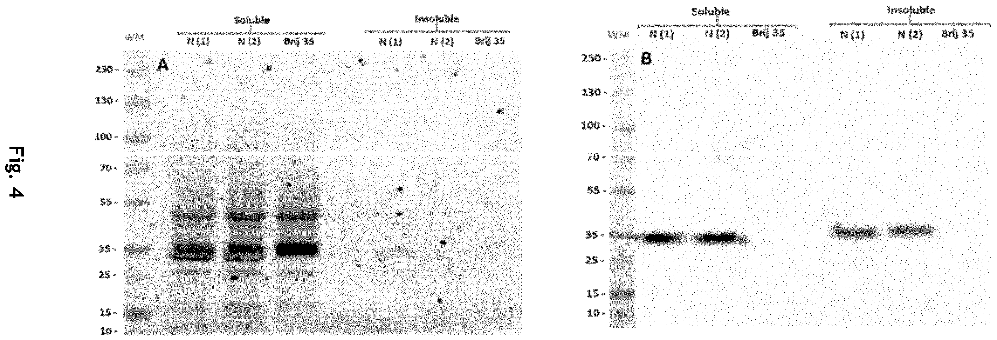

**Figure 4****.** Détection des protéines RHD et MSP en Western-Blot. Analyse des fractions solubles et insolubles des productions de la protéine RHD en présence de nanodisques 50 µM (N1, N2) ou en présence de Brij 35 0.5%. (A) Révélation de la protéine RHD par l'anticorps LOR 15C9, et un anticorps secondaire anti-humain couplé à l'HRP (1/800). (B) La protéine MSP des nanodisques est révélée par un anticorps anti-His couplé à l'HRP (1/10 000). MW correspond au marqueur de poids moléculaire (kDa).

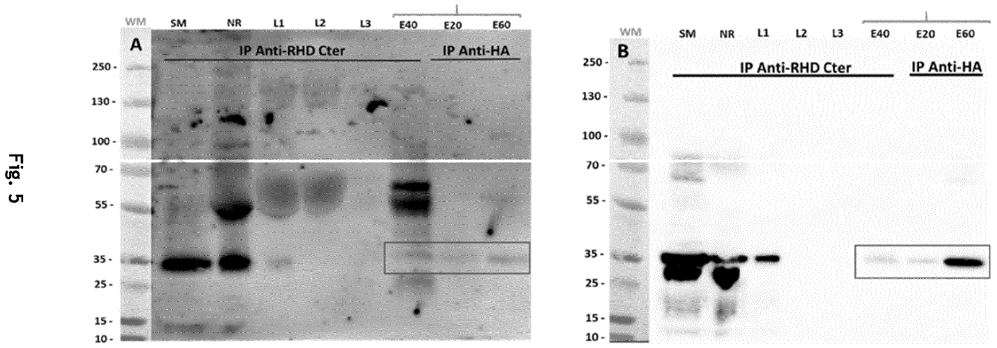

**Figure 5****.** Détection des protéines RHD et MSP par Western-Blot. Analyse des différentes étapes de la purification de la protéine RHD produite en présence de 40 µM de nanodisques (IP Anti-RHD Cter), et comparaison avec les fractions de l'élutions de la purification des protéines RHD produites en présence de 20 et 60 µM (IP Anti-HA). (A) Révélation de la protéine RHD par l'anticorps LOR 15C9, et un anticorps secondaire anti-humain couplé à l'HRP (1/800) (B) La protéine MSP des nanodisques est révélée par un anticorps anti-His couplé à l'HRP (1/10 000). La fraction SM correspond au produit de synthèse in vitro de la protéine RHD, NR : la fraction non retenue par la résine, L1, L2, L3, les fractions des différents lavages, E 20/40/60 : les fractions d'élution, MW, marqueur de poids moléculaire (kDa).

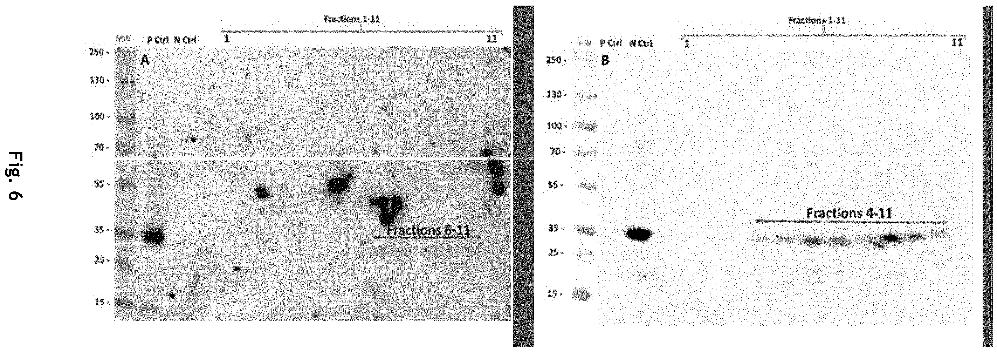

**Figure 6****.Détection** des protéines RHD et MSP par Western-Blot. Analyse des fractions 1-11 du gradient de sucrose (A) Révélation de la protéine RHD par l'anticorps LOR 15C9, et un anticorps secondaire anti-humain couplé à l'HRP (1/800). (B) La protéine MSP des nanodisques est révélée par un anticorps anti-His couplé à l'HRP (1/10 000). MW correspond au marqueur de poids moléculaire (kDa). P Ctrl la protéine RHD produite en présence du Brij 35, N Ctrl : les nanodisques seuls.

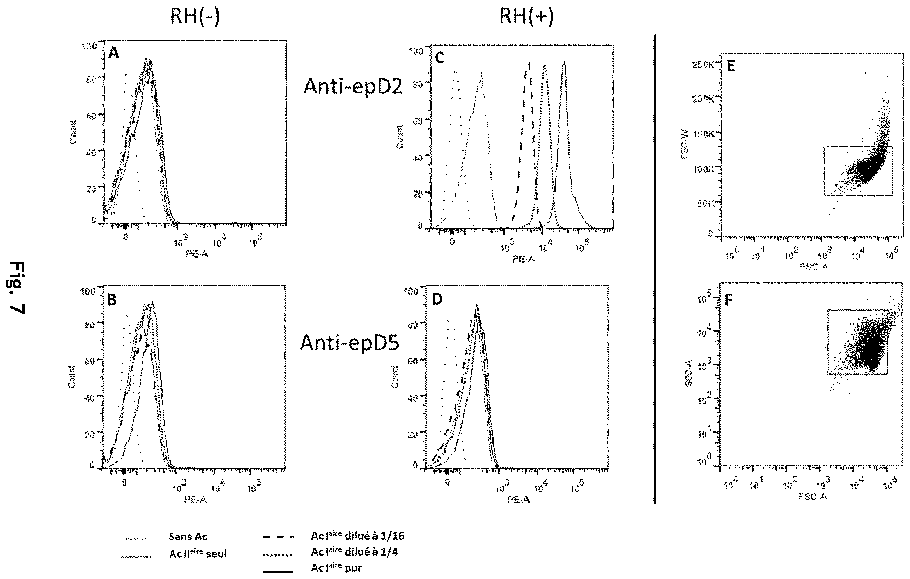

**Figure 7****.** Mise en évidence de la reconnaissance de la protéine RHD par les différents anticorps : Une analyse par cytométrie en flux a été effectuée sur des globules rouges incubés avec différents anticorps. (A)(B) Analyse quantitative de la reconnaissance par les anticorps anti-ep D2 et anti-ep D5 des globules rouges RH (-) utilisés comme contrôle négatif. (C)(D) Analyse quantitative de la reconnaissance des globules rouges RH (+) par les anticorps anti-ep D2 et anti-ep D5. (E)(F) Sélection des populations et sous populations étudiées.

### EXEMPLES

### Matériels et méthodes

### I. Matériel

Le Kit RTS 100 *E. coli* HY conservé à -20°C est fourni par 5 PRIME. Les nanodisques MSP1E3D1-His _ POPC (500 µM) conservés à -80°C viennent de Cube Biotech. Les plaques ELISA utilisées sont de type Nunc MaxiSorp 96 puits (Dutscher) et les plaques du FACS de Corning. La résine protéine A sépharose 4B provient de GE Healthcare Life Sciences. La résine Agarose HA ainsi que les détergents (C₁₂E₈, Brij 35, Brij 58) à 20% et conservés à -20°C proviennent de Sigma-Aldrich.

L'anticorps polyclonal de lapin MPC8 reconnait les résidus 408-416 de la partie C terminale de la protéine RHD (Apoil et al., 1997), tandis que l'anticorps LOR 15C9 est un anticorps monoclonal (Apoil et al., 1997) qui reconnait les résidus 320-331 et 350-354 de la protéine RHD. Les anticorps secondaires couplés à l'HRP (Horse Radish Peroxidase) dirigés contre des IgG humains proviennent de P.A.R.I.S (Western Blot) et de Jackson (ELISA). Un anticorps murin Anti Penta-His conjugué à l'HRP est fourni avec le kit (RGS·His HRP Conjugate Kit) de Qiagen. Un anticorps de chèvre anti-IgG humains est couplé à la R-Phycoérythrine (PE) (Beckman).

Un vecteur personnalisé pIVEX-HA a été développé pour l'expression de la protéine RHD dans un système acellulaire. Il a été généré en substituant le tag His du vecteur pIVEX2.3d (5 PRIME) par un tag HA, ce qui permet d'obtenir une protéine fusionnée à un tag-HA en C-terminal.

### II. Méthodes

### II.1. Expression acellulaire de la protéine

Le principe de l'expression *in vitro* des protéines est d'introduire l'ADN plasmidique contenant le cadre ouvert de lecture, codant la protéine d'intérêt, dans un milieu réactionnel contenant les éléments nécessaires à la synthèse de la protéine. Toute la machinerie transcriptionnelle et traductionnelle est apportée par le lysat cellulaire S30 *d'E.coli.*

Deux types de systèmes ont été utilisés. Un premier système où tous les composants sont mélangés dans un seul compartiment (méthode en « batch ») est utilisé dans le cadre d'un criblage des différentes conditions d'expression. Un deuxième système est le CECF *(Continous Exchange Cell Free)* (Shirokov et al., 2007), où la synthèse de la protéine a lieu dans un compartiment, séparé d'un réservoir par une membrane semi-perméable, permettant de diluer les déchets produits et de fournir les substrats. Le système CECF a été utilisé pour la production de la protéine à grande échelle car l'expression de la protéine est continu pouvant aller jusqu'à 24h avec un rendement meilleur en comparaison avec le système en « batch ».

### II.1.A Expression de la protéine en système « Batch »

Le protocole expérimental développé par la société 5PRIME permet de synthétiser la protéine à petite échelle dans un volume de 50 µl dans le kit RTS 100 *E. coli* HY. Le kit RTS 100 et les nanodisques MSP1E3D1-His_POPC (500 µM) ou le détergent 20% (C₁₂E₈, Brij 35, Brij 58) sont mis à décongeler sur la glace. Le mélange réactionnel (Lysat E.coli 12 µl ; Mix énergétique 10 µl ; Acides aminés 12 µl ; Méthionine 1 µl ; Tampon de reconstitution 5 µl) nécessaire pour la production de la protéine RhD en présence de nanodisques est préparé dans un tube Eppendorf à température ambiante. Le mélange de 50 µl est ensuite incubé en agitation dans un thermomixer Eppendorf à 30°C, 750 rpm pendant 5 heures.

Après expression, le mélange est centrifugé pendant 10 minutes à 22000g à 4°C pour séparer la fraction soluble (le surnageant) de la fraction insoluble (le culot).

### II.1.B Expression de la protéine en système CECF

Le système CECF a été utilisé pour la production de la protéine en présence de détergent ou nanodisques dans un grand volume (1-2 ml). Les réactions ont été réalisées dans les proportions indiquées en annexe.

### II.2. Purification

### II.2.A Immunoprécipitation avec l'agarose HA

Pour immunoprécipiter la protéine RHD-HA produite en présence de détergent **(condition 1)** ou de nanodisques **(condition** 2) **(tableau 1),** 1 ml de la fraction soluble de la production *in vitro* est ajouté à 250 µl (1 CV) de billes d'agarose anti-HA prélavées avec du tampon A₁ ou A₂. Le tube est incubé sous rotation à 4° C sur la nuit. La fraction non retenue est ensuite récupérée par centrifugation à 2000g pendant 10 minutes à 4°C. Par la suite, plusieurs lavages sont effectués avec du tampon A₁ ou A₂ (18 CV) puis le tampon B₁ ou B₂ (28 CV). Pour effectuer les lavages, les tubes sont centrifugés à 5200g pendant 5 min à 4°C, et le surnageant est retiré. Après le dernier lavage, les billes auxquelles les protéines sont liées sont resuspendues et incubées en rotation pendant 4 heures à 4° C, dans 325 µl de tampon d'élution C₁ ou C₂. L'éluat est récupéré après centrifugation pendant 15 min à 18000g 4° C.

### II.2.B Immunoprécipitation avec la protéine A sépharose 4B

Sur la glace, dans un tube Eppendorf, 30 µl de la fraction soluble de la production *in vitro* est diluée à 1/5^{ème} dans du tampon A₃ (tableau 3) puis incubée avec 10 µl de l'anticorps MPC8 purifié (1.5 mg/ml) sur la nuit à 4°C sur la roue. Le complexe formé est ensuite incubé pendant 1 heure à 4°C, sur la roue avec 50 µl de résine protéine A sépharose 4B préalablement lavée avec du tampon A₃.

La fraction non retenue est récupérée après centrifugation à 15000g pendant 5 minutes à 4° C avec une faible décélération. Le culot est ensuite lavé 3 fois par centrifugation avec 1 ml de tampon A₃, puis 2 lavages avec du tampon B₃, et un dernier lavage avec du tampon C₃. Pour éluer la protéine, la résine est chauffée pendant 5 minutes à 100° C avec 50 µl de Laemmli 2X. Après centrifugation à 15000g pendant 5 minutes, l'éluat est récupéré.

**Tableau 1. Tampons utilisés pour la purification de la protéine RHD.**

| | **Agarose HA** | | **Protéine A Sépharose 4B** |
|---|---|---|---|
| | **Protéine en présence de détergent** | **Protéine en présence de nanodisques** | **Protéine en présence de nanodisques** |
| **Tampons A₁, A₂, A₃** | 10 mM Hepes, 150 mM NaCl, pH 6.8 (C12E8 0.3%) | 50 mM TrisHCl, 150 mM NaCl, pH 7.4 | PBS, BSA 0.5%, 5 mM EDTA |
| **Tampons B₁, B₂, B₃** | 10 mM Hepes, 50 mM K2SO4, pH 6.8 (C12E8 0.3%) | 20 mM Tris HCl, 100 mM NaCl, pH 7.4 | PBS, BSA 0.5%, 5 mM EDTA, 10 mMNaCl |
| **Tampons C₁, C₂, C₃** | TpB + peptide HA (5mg/1300µl) glycerol 1% pH 6.8 | TpB, peptide HA(5 mg/1300µl) pH 6.8 | PBS, 5 mM EDTA |

### II.3. Gradient de sucrose

Le produit de synthèse a été déposé sur un gradient de sucrose discontinu (5-10-15-20-30 % dans du PBS), suivi d'une ultracentrifugation à 210000g pendant 18h à 4°C, en utilisant les conditions décrites par T H. Bayburt *et al,2007.* Les fractions de 0,5 ml ont été recueillies de haut en bas et analysées par Western-Blot.

### II.4. Electrophorèse

Les échantillons sont dénaturés dans le tampon de charge (Tris-HCl 5 mM, sucrose 8.56%, SDS 1%, 5% β-mercaptoéthanol) et déposés sur un gel de polyacrylamide dénaturant à 10% ou en gradient 4-12% Bis-Tris polyacrylamide (Invitrogen). Les protéines sont séparées en fonction de leurs poids moléculaires à 180V par électrophorèse dans le tampon de migration (25 mM Tris-HCl, 192 mM Glycine, 0,1% SDS ou 50 mM MOPS, 50 mM Tris HCl, 0.1% SDS, 1 mM EDTA).

### II.5. Coloration au nitrate d'argent

Après incubation dans un tampon de fixation (50% Méthanol, 12% Acide acétique, 0.05% Formaldéhyde) en agitation pendant 1 heure, suivi de plusieurs rinçages en Ethanol 50 %, le gel est traité avec une solution de Thiosulfate de sodium 1% pendant 1 minute. Après plusieurs rinçages dans l'eau, il est mis à incuber pendant 20 min dans le mélange de coloration (AgNO₃ 0.2%, Formaldéhyde 0.075 %). Après 2 rinçages dans l'eau, une solution de révélation (0.05% Formaldéhyde, 0.04% Thiosulfate, Na₂CO₃ 6%), fait apparaître les protéines sur le gel. La réaction est stoppée par une solution d'acide acétique 10% pendant 30 minutes.

### II.6. Western-Blot

Une fois la migration terminée, les protéines sont transférées sur une membrane de nitrocellulose (Amersham) pendant 2 heures à 30V dans un tampon de transfert (12,5 mM Tris-HCl, 96 mM Glycine, 20% Ethanol).

### II.6.A Révélation de la protéine RHD

Après transfert, la membrane est lavée une fois avec du PBS seul puis saturée dans une solution de PBS contenant du lait dilué à 5% pendant 1 heure à température ambiante sous agitation. La membrane est ensuite incubée avec l'anticorps primaire LOR 15C9 pendant une nuit à 4° C sur une plaque rotative.

Après plusieurs lavages en PBS-Tween20 0,1%, la membrane est incubée pendant 1 heure en présence de l'anticorps secondaire murin anti-IgG humains couplé à l'HRP dilué au 1/800^{ème} dans du PBS lait 5% sous agitation. Après plusieurs lavages en PBS-Tween20 0,1% puis PBS seul, la réaction enzymatique est révélée par chimioluminescence (GE Healthcare Life Sciences).

### II.6.B Révélation de la protéine MSP des nanodisques

La protéine MSP des nanodisques, qui porte un Tag Poly-Histidine, est révélée en Western-Blot par l'anticorps Anti-His conjugué à l'HRP (Qiagen).

Après transfert, la membrane est rincée 2 fois avec du TBS seul puis bloquée pendant 1 heure à température ambiante dans la solution de blocage préparée extemporanément selon le protocole fourni avec le kit. Après 2 lavages en TBS-Tween20 0.05% à température ambiante et un dernier lavage en TBS seul, la membrane est incubée pendant 1 heure à température ambiante avec l'anticorps anti Penta-His-HRP dilué à 1/10000^{ème} dans la solution de blocage. Après plusieurs lavages en TBS-Tween20 0.05% puis le TBS seul, la réaction enzymatique est révélée par chimioluminescence.

### II.7. Cytométrie en flux

La reconnaissance de la protéine RHD par différents anticorps monoclonaux humains anti-epD est révélée par marquage indirect sur des globules rouges.

Pour cela, 0,5 µL de culot globulaire (5.10⁶ cellules) est suspendu dans 1 ml de PBS-BSA 0.2% puis réparti dans les différents puits d'une plaque, à raison de 5.10⁵ cellules/puits. La plaque est centrifugée 3 minutes à 100 g à 4°C puis le surnageant est enlevé.

Après le premier lavage, les globules rouges sont incubés 1h à 4° C avec les anticorps dirigés contre la protéine RHD. Les cellules sont ensuite lavées 2 fois en PBS 0.2% BSA et incubées à l'abri de la lumière pendant 1h à 4°C avec un anticorps anti-IgG humains couplé à la R-Phycoérythrine (PE) dilué à 1/100^{ème} dans du PBS 0.2% BSA. Après 3 lavages en PBS 0.2% BSA, les cellules sont resuspendues dans 200 µl de PBS et analysées en cytométrie en flux (BD FCS Canto II, BD Biosciences).

Les résultats sont analysés par le logiciel FlowJo.

### II.8. Test ELISA

Pour révéler l'interaction entre les différents anticorps et la protéine d'intérêt, un test ELISA sandwich a été réalisé.

L'anticorps de capture MPC8 est mis en incubation dans les puits en PBS à raison de 1ng/µl par puits. La capture se fait sur la nuit à température ambiante. Après plusieurs lavages en PBS, les puits sont ensuite saturés par du PBS-lait 5% puis rincés par du PBS.

La protéine RHD produite *in vitro* en présence de nanodisques est alors ajoutée pendant 45 minutes à 37°C dans les puits. Après rinçage en PBS, les anticorps humains anti-RHD à tester sont incubés avec ce complexe pendant 45 minutes à 37° C, le surplus est éliminé par des lavages successifs.

Les complexes formés sont détectés grâce à un anticorps anti-anti-IgG humains (1/50000^{ème} dans du PBS), épuisé contre les IgG de souris et de lapin et couplé à l'HRP. L'ensemble est incubé à 37°C pendant 30 minutes. Après lavage, le complexe lié à l'enzyme est révélé par l'ajout du TMB (Bio-Rad) dans les puits. La réaction est stoppée par une solution d'H₂SO₄ 0,1N et l'absorbance est mesurée à une longueur d'onde de 450 nm.

### Résultats

### I. Test d'expression et de solubilité de la protéine RHD en présence de détergent

Nous avons étudié la compatibilité de différents détergents avec la production de la protéine RHD dans le système de traduction *in vitro.*

Trois détergents non-ioniques sont utilisés, C₁₂E₈, Brij 35, Brij 58. Le choix de ces détergents a été effectué en fonction de leur caractère non dénaturant sur les protéines membranaires. La concentration utilisée a été choisie en tenant compte de leur basse concentration micellaire critique (CMC, concentration à partir de laquelle des micelles se forment), (0,11 % pour le Brij 35, 0,0086% pour le Brij 58 et 0.006% pour le C₁₂E₈). Une analyse par Western-Blot des productions en « Batch » de la protéine RHD en présence des 3 détergents (C₁₂E₈ 0,5%, Brij 35 0,5%, Brij 58 0,5 %) (Figure 1) a montré la présence de la protéine uniquement dans les fractions solubles pour les réactions contenant du Brij 35 ou du Brij58. Pour la réaction contenant du C₁₂E₈, seul un signal de faible intensité a été détecté dans la fraction insoluble, alors qu'en absence du détergent, la protéine est majoritairement dans le culot (la fraction insoluble). Le C₁₂E₈ n'est donc pas favorable à l'expression de la protéine RHD lors d'une synthèse *in vitro.* Le Brij 35 s'avère ainsi le détergent le plus compatible pour la production de la protéine RHD. De ce fait, c'est le détergent choisi pour produire la protéine à plus grande échelle.

### I.1. Production à grande échelle et purification de la protéine RHD en présence de détergent

Une fois les conditions optimales d'expression et de solubilité établies, une production à grande échelle dans 2 ml de volume réactionnel est réalisée en utilisant le système CECF en présence du détergent choisi (Brij 35 0,5%). La fraction soluble est purifiée sur la base de la reconnaissance, par une résine Agarose-HA, du tag HA fusionné à la protéine. L'élution est réalisée par compétition avec le peptide HA. L'ensemble de la purification est effectué en substituant le Brij 35 par le C₁₂E₈ 0.3%, détergent précédemment utilisé avec succès par l'équipe pour la purification et la reconstitution fonctionnelle dans des liposomes de la protéine RHCG (Mouro-Chanteloup et al., 2010), une protéine RH homologue non érythrocytaire.

Les résultats illustrés sur **la** **figure 2** indiquent que la protéine RHD soluble se retrouve essentiellement dans l'éluat et le lavage d'élution. On constate également qu'une partie de la protéine n'est pas fixée sur la résine, et se retrouve dans la fraction non retenue (NR). L'analyse de l'éluat en nitrate d'argent montre qu'il ne contient que la protéine RHD. Le temps de réaction sélectionné pour la production de la protéine RHD soluble est de 8 heures de réaction car à partir de 16 heures de réaction, il existe un risque d'agrégation de la protéine RHD.

### II. Test d'expression et de solubilité de la protéine RHD en présence de nanodisques

Nous avons étudié l'effet des nanodisques sur le niveau d'expression de la protéine RHD. La protéine est produite en présence de différentes concentrations de nanodisques (e.g. MSP1E3D1-His_POPC (20, 40, 60, 80 µM).

Les produits de synthèse de chaque production sont analysés par Western-Blot. De même que pour la protéine produite en présence du Brij 35 0,5%, la majeure partie de la protéine produite en présence de nanodisques est localisée dans les fractions solubles **(****Figure 3****).** La présence de nanodisques à des concentrations de 20, 40, 60 µM permettent d'obtenir des rendements de synthèse aussi élevé qu'en présence du Brij 35. En revanche, la présence d'une grande concentration de nanodisques (80 µM) entraine une synthèse diminuée. Comme attendu, l'intensité du signal de la protéine MSP dans la fraction soluble augmente avec la concentration des nanodisques. On note également que les profils de migration des fractions solubles et insolubles sont différents, cette différence est due à la présence de polymères dans le lysat *d'E.coli* comme signalé dans le protocole fourni avec le kit RTS 100.

### II.1 Production à grande échelle de la protéine RHD en présence de nanodisques (CECF)

Deux productions à grande échelle ont été réalisées dans un volume final de 1 ml en présence de nanodisques à une concentration de 50 µM. Cette concentration semble, selon les tests d'expression à petite échelle, adéquate pour la production de la protéine RHD avec un bon rendement. Une production contrôle en présence de Brij 35 0.5% a été réalisée en parallèle. L'analyse des produits de synthèse par Western-Blot montre que la protéine RHD produite est essentiellement dans la fraction soluble ainsi que les nanodisques. On remarque un dépôt plus important des nanodisques dans la fraction insoluble suggérant la formation d'agrégats **(****Figure 4****).**

### III. Différentes approches de purification de la protéine RHD produite en présence de nanodisques

De même que pour la production en présence de détergent, la protéine produite en présence de nanodisques se retrouve dans la fraction soluble contenant également plusieurs protéines de lysat bactérien *d'E.coli.* Sa purification est donc nécessaire, ainsi que la séparation des nanodisques pleins (dans lesquels la protéine a pu s'insérer) des nanodisques vides. Deux approches ont été utilisées pour purifier la protéine RHD insérée dans des nanodisques. La première approche consiste à immunoprécipiter la protéine RHD en utilisant 2 protocoles, la deuxième approche est l'ultra-centrifugation en gradient de sucrose du produit de synthèse (Bayburt et al., 2007).

Le premier procédé d'immuno-précipitation testé consiste à retenir la protéine RHD fusionnée à un tag HA avec une résine agarose HA. L'élution est réalisée en présence de peptide HA, qui détache la protéine RHD par compétition.

Dans le deuxième protocole, la protéine est immuno-précipitée en utilisant une résine protéine A sépharose 4B. La production *in vitro* est mise en contact avec un anticorps polyclonal de lapin anti-RH (MPC8), le complexe est ensuite incubé avec la résine protéine A Sépharose 4B qui fixe l'anticorps complexé à la protéine. A la fin, la protéine est éluée par chauffage en présence du tampon Laemmli.

Dans les fractions d'élution des 2 protocoles, les nanodisques ont été co-élués avec la protéine RHD, bien qu'il y ait un faible rendement avec une perte au cours des différentes étapes de la purification comme observé par Western-Blot **(****Figure 5****).** Selon les résultats des immuno-précipitations, la protéine RHD et les nanodisques se retrouvent dans l'éluat, ce qui indiquerait une insertion de la protéine dans les nanodisques.

Ce résultat a été également retrouvé par une analyse en gradient de sucrose du produit de synthèse. Les échantillons provenant des 11 premières fractions ont été recueillies de haut en bas puis analysées par Western-Blot **(****Figure 6****).** On constate que la protéine RHD et la protéine MSP des nanodisques sont dans les mêmes fractions 6-11, qui correspondent à une concentration de 10-15% de sucrose, alors que les nanodisques vides qui sont minoritaires se retrouvent dans les fractions plus hautes (4-5).

### IV. Analyse immunologique des antigènes de la protéine RHD produite en présence de nanodisques

Afin de vérifier la conformation de la protéine traduite *in vitro* en présence de nanodisques, nous souhaitons développer un test « ELISA sandwich » en utilisant des anticorps monoclonaux conformationnels (anti-ep D) dirigés contre les 9 épitopes D, selon la classification de Tippett. Ces anticorps ne reconnaissent la protéine que sous sa forme native, et donc leur réactivité dépend de la conformation de celle-ci. L'anticorps non conformationnel LOR15C9 est aussi utilisé dans ce test comme contrôle positif.

### IV.1. Test des anticorps par cytométrie en flux

Avant la mise au point de ce test ELISA, des surnageants provenant de laboratoires différents et contenant les anticorps anti-ep D dont la concentration est inconnue, ont été analysés par cytométrie en flux sur des globules rouges exprimant la protéine RHD.

Plusieurs dilutions ont été testés (pur, 1/4, 1/16) et des globules rouges RHD (-) ont été utilisés comme contrôle négatif **(****Figure 7 A-B).**

Les résultats montrent que les anticorps conformationnels ou non, reconnaissent les différents épitopes de la protéine RHD en fonction de leurs concentrations **(****Figure 7 C).** L'analyse a été réalisée dans les sous populations « single cells » afin d'éliminer les cellules en amas. Les résultats montrent aussi une grande homogénéité au sein de l'échantillon étudié **(****Figure 7 E-F).**

### V. Mise au point du test ELISA

Après la détermination de la dilution à laquelle les anticorps vont être utilisés pour réaliser un test ELISA, il faut également optimiser d'autres paramètres tels que le choix de l'anticorps de capture et le blocage de la plaque.

Comme anticorps de capture, nous avons le choix entre l'anti-HA et MPC8. Toutefois l'anti-HA ne peut être utilisé que sur des fractions brutes et non sur la protéine purifiée par immuno-précipitation avec l'agarose HA. En effet la présence du peptide HA dans la fraction d'élution peut diminuer grandement la sensibilité du test. De ce fait, on a retenu le MPC8 comme anticorps de capture, et qui peut être utilisé avec toutes les fractions brute ou purifiée.

Différentes dilutions du produit synthétisé en présence de 40 µM de nanodisques ont été utilisées pour un premier test ELISA (1/40, 1/80, 1/160) en duplicata, avec les différentes conditions mis au point précédemment, en utilisant l'anticorps LOR15C9 comme anticorps primaire au 1/32^{ème} **(tableau 2),** et un anticorps anti-humain conjugué. Des puits-contrôle ont été réalisés en absence de la protéine ou de l'anticorps primaire LOR 15C9.

Sur **le tableau 2,** l'intensité du signal dans les puits-tests augmente en fonction de la concentration de la protéine. Cette intensité est supérieure à celle des puits-contrôle malgré le bruit de fond.

**Tableau 2. Résultats du test ELISA de différentes dilutions du produit de synthèse de la protéine RHD insérée dans des nanodisques.**

| | **Puits-Contrôles** | | **Puits-tests** | | | | | |
|---|---|---|---|---|---|---|---|---|
| | **Protéine (-)** | **Ac l^{aire} (-)** | **Protéine 1/40** | | **Protéine 1/80** | | **Protéine 1/160** | |
| **DO 450 nm** | 0,129 | 0,143 | 0,2 | 0,247 | 0,214 | 0,208 | 0,124 | 0,143 |

### Références bibliographiques

Apoil, P. A., M. E. Reid, G. Halverson, I. Mouro, Y. Colin, F. Roubinet, J. P. Cartron, and A. Blancher.1997. A human monoclonal anti-D antibody which detects a nonconformation-dependent epitope on the RhD protein by immunoblot. Br J Haematol 98: 365-374.
Avent, N.D., and M.E. Reid. 2000, The Rh blood group system: a review. Blood 95(2):375-387,
Bayburt, T. H., A. J. Leitz, G. Xie, D. D. Oprian, and S. G. Sligar. 2007. Transducin activation by nanoscale lipid bilayers containing one and two rhodopsins. J Biol Chem 282:14875-14881**.**
Carlson, E.D., R. Gan, C.E. Hodgman, and M.C. Jewett. 2012 Cell-Free Protein Synthesis: Applications Come of Age, Biotechnol Adv. 30(5): 1185-1194.
Cartron, J. P. 1999. RH blood group system and molecular basis of Rh-deficiency. Baillieres Best Pract Res Clin Haematol 12:655-689.
Cartron, J. P., C. Rouillac, C. Le Van Kim, I. Mouro, and Y. Colin. 1996. Tentative model for the mapping of D epitopes on the RhD polypeptide. Transfus Clin Biol 3:497-503.
Denisov, I. G., Y. V. Grinkova, A. A. Lazarides, and S. G. Sligar. 2004. Directed self-assembly of monodisperse phospholipid bilayer Nanodiscs with controlled size. J Am Chem Soc 126:3477-3487.
Goossens D, da Silva N, Metral S, Cortes U, Callebaut I, Picot J, Mouro-Chanteloup I, Cartron JP. 2013. Mice expressing RHAG and RHD human blood group genes. PLoS One 8(11):e80460.Grinkova, Y.V., I.G. Denisov, and S.G. Sligar. 2010 Engineering extended membrane scaffold proteins for self-assembly of soluble nanoscale lipid bilayers. Protein Eng Des Sel. 23(11): 843-848.
Mouro-Chanteloup, I., S. Cochet, M. Chami, S. Genetet, N. Zidi-Yahiaoui, A. Engel, Y. Colin, O. Bertrand, and P. Ripoche. 2010. Functional reconstitution into liposomes of purified human RhCG ammonia channel. PLoS One 5:e8921.
Mouro-Chanteloup, I., A. M. D'Ambrosio, P. Gane, C. Le Van Kim, V. Raynal, D. Dhermy, J. P. Cartron, and Y. Colin. 2002. Cell-surface expression of RhD blood group polypeptide is posttranscriptionally regulated by the RhAG glycoprotein. Blood 100:1038-1047.
Mouro, I., Y. Colin, B. Cherif-Zahar, J. P. Cartron, and C. Le Van Kim. 1993. Molecular genetic basis of the human Rhesus blood group system. Nat Genet 5:62-65.
Rogé, J., and J.M. Betton. 2005 Use of pIVEX plasmids for protein overproduction in Escherichia coli, Microb Cell Fact. 4: 18.
Shirokov, V. A., A. Kommer, V. A. Kolb, and A. S. Spirin. 2007. Continuous-exchange protein-synthesizing systems. Methods Mol Biol 375:19-55.
Tippett, P., C. Lomas-Francis, and M. Wallace. 1996. The Rh antigen D: partial D antigens and associated low incidence antigens. Vox Sang 70:123-131.

## Revendications

1. Procédé de synthèse d'une protéine érythrocytaire choisie parmi RhD, RhCE, RhAG et UTB, ou d'un variant de celle-ci, ledit procédé comprenant les étapes suivantes :
a) la mise en contact d'un acide nucléique codant ladite protéine ou un homologue ayant au moins 95% d'identité avec celle-ci avec un système acellulaire de production de protéines, en présence d'au moins un détergent non-ionique, de liposomes ou de nanodisques ; et
b) la synthèse de ladite protéine.

2. Procédé selon la revendication 1, **caractérisé en ce que** la mise en contact selon l'étape a) se fait en présence d'au moins un détergent non-ionique sélectionné parmi le Brij 35 ou le Brij 58, ou de nanodisques comprenant une protéine MSP choisie parmi les protéines de SEQ ID NO. 9, 10 ou 11.

3. Procédé selon l'une quelconque des revendications 1 ou 2, **caractérisé en ce que** le système acellulaire de production de protéines est un système en batch ou un système acellulaire en échange continu.

4. Procédé selon l'une quelconque des revendications 1 à 3, **caractérisé en ce que** la concentration en nanodisques est inférieure à 80 µM.

5. Procédé selon l'une quelconque des revendications 1 à 4, **caractérisé en ce que** ladite protéine érythrocytaire, ou son variant, et/ou une protéine MSP choisie parmi les protéines de SEQ ID NO. 9, 10 ou 11, est fusionnée à une séquence d'étiquetage.

6. Particule en forme de protéoliposome ou de nanodisque comprenant une protéine érythrocytaire et des lipides susceptible d'être obtenue par le procédé de l'une quelconque des revendications 1 à 5.

7. Particule selon la revendication 6 **caractérisée en ce que** ladite protéine érythrocytaire est choisie parmi RhD, RhCE, RhAG et UTB, ou un homologue ayant au moins 95% d'identité avec celle-ci, et est couplée à un support solide.

8. Composition comprenant la particule selon la revendication 6.

9. Composition selon la revendication 8 comprenant en outre une protéine MSP choisie parmi les protéines de SEQ ID NO. 9, 10 ou 11.

10. Composition selon la revendication 8 ou 9 comprenant une protéine érythrocytaire à une concentration supérieure à 0,01 mg/ml, de préférence supérieure à 0,1 mg/ml.

11. Utilisation de la composition selon l'une quelconque des revendications 8 à 10 dans un test in vitro de détection d'allo-anticorps.

## Patentansprüche

1. Verfahren zur Synthese eines Erythrozytenproteins, ausgewählt aus RhD, RhCE, RhAG und UTB, oder einer Variante davon, wobei das Verfahren die folgenden Schritte umfasst:
a) das Inkontaktbringen einer Nukleinsäure, die für das Protein oder ein Homolog mit mindestens 95% Identität dazu kodiert, mit einem zellfreien Proteinproduktionssystem in Gegenwart von mindestens einem nichtionischen Detergens, von Liposomen oder von Nanodisks; und
b) die Synthese des Proteins.

2. Verfahren nach Anspruch 1, **dadurch gekennzeichnet, dass** das Inkontaktbringen nach Schritt a) in Gegenwart von mindestens einem nichtionischen Detergens, ausgewählt aus Brij 35 oder Brij 58, oder von Nanodisks erfolgt, die ein MSP-Protein enthalten, das aus den Proteinen der SEQ ID NO. 9, 10 oder 11 ausgewählt ist.

3. Verfahren nach einem der Ansprüche 1 oder 2, **dadurch gekennzeichnet, dass** das zellfreie Proteinproduktionssystem ein Batch-System oder ein zellfreies System mit kontinuierlichem Austausch ist.

4. Verfahren nach einem der Ansprüche 1 bis 3, **dadurch gekennzeichnet, dass** die Nanodisks-Konzentration unter 80 µM liegt.

5. Verfahren nach einem der Ansprüche 1 bis 4, **dadurch gekennzeichnet, dass** das Erythrozytenprotein oder dessen Variante und/oder ein MSP-Protein, ausgewählt aus den Proteinen der SEQ ID NO. 9, 10 oder 11, mit einer Markersequenz fusioniert ist.

6. Partikel in Form von Proteoliposom oder von Nanodisk, umfassend ein Erythrozytenprotein und Lipide, die durch das Verfahren nach einem der Ansprüche 1 bis 5 erhaltbar sind.

7. Partikel nach Anspruch 6, **dadurch gekennzeichnet, dass** das Erythrozytenprotein aus RhD, RhCE, RhAG und UTB oder einem Homolog mit mindestens 95% Identität dazu ausgewählt ist und an einen festen Träger gekoppelt ist.

8. Zusammensetzung, umfassend den Partikel nach Anspruch 6.

9. Zusammensetzung nach Anspruch 8, umfassend ferner ein MSP-Protein, ausgewählt aus den Proteinen der SEQ ID NO. 9, 10 oder 11.

10. Zusammensetzung nach Anspruch 8 oder 9, umfassend ein Erythrozytenprotein in einer Konzentration von mehr als 0,01 mg/ml, vorzugsweise von mehr als 0,1 mg/ml.

11. Verwendung der Zusammensetzung nach einem der Ansprüche 8 bis 10 in einem In-vitro-Test zum Nachweis von Alloantikörpern.

## Claims

1. A method of synthesizing an erythrocyte protein selected from RhD, RhCE, RhAG and UTB, or a variant thereof, said method comprising the steps of:
a) contacting a nucleic acid encoding said protein or a homolog having at least 95% identity therewith with an acellular protein production system, in the presence of at least one non-ionic detergent, liposomes or nanodiscs; and
b) synthesis of said protein.

2. Method according to claim 1, **characterised in that** the contacting according to step a) occurs in the presence of at least one non-ionic detergent selected from Brij 35 or Brij 58, or nanodiscs comprising a MSP protein selected from proteins of SEQ ID NO. 9, 10 or 11.

3. Method according to any one of claims 1 or 2, **characterised in that** the acellular system for protein production is a batch system or an acellular system with continuous exchange.

4. Method according to any one of claims 1 to 3, **characterised in that** the concentration in nanodiscs is less than 80 µM.

5. Method according to any one of claims 1 to 4, **characterised in that** said erythrocyte protein, or variant thereof, and/or a MSP protein selected from proteins of SEQ ID NO. 9, 10 or 11, is fused to a labelling sequence.

6. A proteoliposome- or nanodisk-shaped particle comprising an erythrocyte protein and lipids, obtainable by the method of any of claims 1 to 5.

7. A particle according to claim 6, **characterized in that** said erythrocyte protein is selected from RhD, RhCE, RhAG, and UTB, or a homolog having at least 95% identity thereto, and is coupled to a solid support.

8. A composition comprising the particle according to claim 6.

9. A composition according to claim 8 further comprising an MSP protein selected from the proteins of SEQ ID NO. 9, 10, or 11.

10. A composition according to claim 8 or 9 comprising an erythrocyte protein at a concentration greater than 0.01 mg/ml, preferably greater than 0.1 mg/ml.

11. Use of the composition according to any one of claims 8 to 10 in an in vitro alloantibody detection assay.
